# EUROPÄISCHE PATENTSCHRIFT

(11) **EP 1 434 557 B1**
(45) Veröffentlichungstag und Bekanntmachung des Hinweises auf die Patenterteilung: **09.11.2005**
(21) Anmeldenummer: 02777201.1
(22) Anmeldetag: 25.09.2002
(51) Int. Cl.: A61K 7/075

(54) **MITTEL ZUM FÄRBEN VON KERATINHALTIGEN FASERN**
AGENT FOR THE COLORATION OF KERATIN-CONTAINING FIBERS
AGENT DE COLORATION DES FIBRES KERATINIQUES

(30) Priorität: 04.10.2001 DE 10148847
(43) Veröffentlichungstag der Anmeldung: 07.07.2004
(73) Patentinhaber: Henkel Kommanditgesellschaft auf Aktien, 40589 Düsseldorf (DE)
(72) Erfinder: MÖLLER, Hinrich, 40789 Monheim (DE); OBERKOBUSCH, Doris, 40591 Düsseldorf (DE)
(86) Internationale Anmeldenummer: PCT/EP2002/010730
(87) Internationale Veröffentlichungsnummer: WO 2003/030845

(56) Entgegenhaltungen:
- EP-A- 0 951 900
- WO-A-01/34106
- FR-A- 2 018 056
- GB-A- 1 213 697

## Beschreibung

Die Erfindung betrifft ein Mittel zum Färben von keratinhaltigen Fasern, insbesondere menschlichen Haaren, das aromatische oder heteroaromatische Aldehyde und/oder Ketone in Kombination mit 4-Aminopyrazolin-5-onen enthält, die Verwendung dieser Kombination in Mitteln zum Färben von keratinhaltigen Fasern sowie ein Verfahren zum Färben von keratinhaltigen Fasern, insbesondere menschlichen Haaren.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2'-hydroxyethyl)-p-phenylendiamin, 2-(2',5'-Diaminophenyl)-ethanol, 2-(2',5'-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, p-Phenylendiamin, 2,4-Diaminophenoxyethanol, 2-Amino-4-(2'-hydroxyethylamino)-anisol (Lehmanns Blau), 1-Phenyl-3-methyl-pyrazol-5-on, 2,4-Dichlor-3-aminophenol, 1,3-Bis-(2',4'-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluss von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, dass die Färbungen häufig nur über unzureichende Echtheitseigenschaften verfügen.

In der DE 196 19 112 A1 werden Mittel zur oxidativen Färbung von Keratinfasem auf der Basis einer Entwicklersubstanz-Kupplersubstanzkombination offenbart, worin als Entwicklersubstanzen 4-Amino-5-hydroxypyrazolderivaten eingesetzt werden.

Aus der EP 0 974 337 ist ein weiteres Mittel zur oxidativen Färbung von Keratinfasern bekannt, das mindestens ein Oxidationsfarbstoffvorprodukt sowie ein 3-Amino-pyrazolin als Kupplersubstanz enthält.

Die Verwendung der unten näher beschriebenen Kombination aus aromatischen oder heteroaromatische Aldehyden und/oder Ketonen und 4-Aminopyrazolin-5-onen zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, dass eine Kombination aus aromatischen oder heteroaromatischen Aldehyden und/oder Ketonen mit der Formel I und den in der Formel IIa bzw. IIb dargestellten 4-Aminopyrazolin-5-onen sich auch in Abwesenheit von oxidierenden Agentien hervorragend zum Färben von keratinhaltigen Fasern eignet. Man erhält Ausfärbungen mit hervorragender Brillanz und Farbtiefe in vielfältigen Farbnuancen. Der Einsatz von oxidierenden Agentien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend
mindestens ein aromatisches oder heteroaromatisches Aldehyd und/oder Keton mit der Formel I oder physiologisch verträgliche Salze davon, 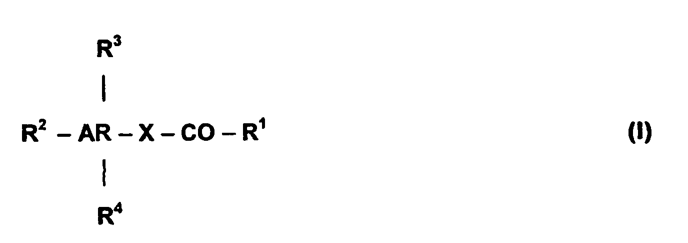 wobei
- AR steht für Benzol, Naphthalin, Pyridin, Pyrimidin, Pyrazin, Pyrazidin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
- R¹ steht für ein Wasserstoffatom, eine C₁-C₄-Alkyl-, C₁-C₅-Acyl-, C₂-C₄-Alkenyl-, C₁-C₄-Perfluoralkyl-, eine ggf. substituierte Aryl- oder Heteroarylgruppe,
- R², R³ und R⁴ stehen unabhängig voneinander für ein Wasserstoffatom, ein Halogenatom, eine C₁-C₄-Alkyl-, C₁₋₄-Alkoxy-, C₁-C₄-Aminoalkyl-, C₁-C₄-Hydroxyalkylgruppe, eine C₁-C₄-Acylgruppe, eine Carboxy-, Carboxylato-, Sulfo-, Sulfato-, C₂-C₆-Alkenyl-, Aryl-, eine Aryl-C₁-C₄-alkylgruppe, eine Hydroxy-, Nitro-, Pyrrolidino-, Morpholino-, Piperidino-, Amino- bzw. Ammonio- oder 1-Imidazol(in)io-gruppe, wobei die letzten drei Gruppen mit C₁₋₄-Alkyl-, C₁₋₄-Carboxyalkyl-, C₁₋₄-Hydroxyalkyl-, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können, durch C₁-C₄-Alkyl-, C₂-C₆-Alkenyl- oder C₁-C₄-Hydroxyalkoxygruppen substituiert sein können, wobei auch zwei der Reste aus R², R³, R⁴ und X-CO-R¹ einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R², R³ und R⁴,
- X steht für eine direkte Bindung, eine Carbonyl-, eine Carboxy-(C₁-C₄)-alkylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei X zusammen mit der CO-R¹-Gruppe auch einen gegebenenfalls substituierten 5-, 6- oder 7-Ring bilden kann,
sowie mindestens ein 4-Aminopyrazolin-5-on mit der Formel (IIa) oder (IIb) oder deren physiologisch verträgliche Salze, 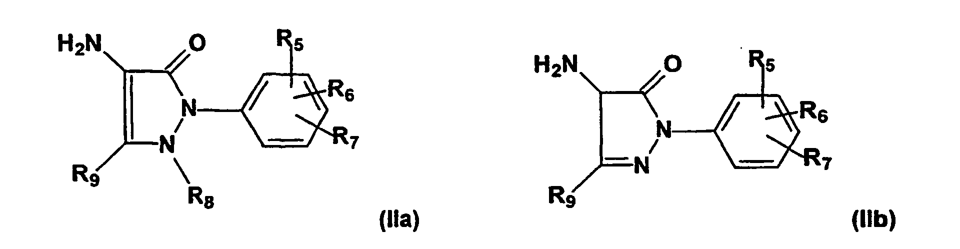 in denen
- R⁵, R⁶ und R⁷ stehen unabhängig voneinander für ein Wasserstoffatom, ein Halogenatom, eine C₁₋₄-Alkyl-, C₁₋₄-Alkoxy- oder C₁₋₄-Hydroxyalkoxygruppe, eine Hydroxy-, Carboxy-, Carboxylato-, C₁₋₄-Alkoxycarbonyl-, gegebenenfalls substituierte Carbamoyl-, Sulfo-, Sulfato-, Sulfamoylgruppe oder Aminogruppe, die durch C₁₋₄-Alkyl- oder Hydroxyalkylgruppen substituiert sein kann,
- R⁸ und R⁹ stehen unabhängig voneinander für ein Wasserstoffatom, eine Hydroxy-, Amino-, C₁₋₄-Alkyl-, C₁₋₄-Alkoxy-, Carbamoyl-, Carboxyl-, C₁₋₄-Alkoxycarbonyl-, Aryl-, Aryl-C₁₋₄-alkyl- oder Heteroarylgruppe.

Die N-Heteroaromaten und Aminogruppen können quaterniert sein, z.B. durch durch C₁-C₄-Alkyl-, Aralkyl-, C₁-C₄-Sulfoalkyl-, C₁-C₄-Carboxyalkyl-, C₁-C₄-Hydroxyalkyl-, C₂-C₆-Alkenylgruppen, die ggf. substituiert sein können, oder eine Oxidogruppe. Als Gegenionen der quatemierten heteroaromatischen Carbonylverbindungen können Halogenide, wie Chlorid, Bromid oder Iodid, Benzolsulfonat, p-Toluolsulfonat, C₁-C₄-Alkansulfonat, wie Methansulfonat oder Ethansulfonat, Trifluormethansulfonat, Perchlorat, Sulfat, Hydrogensulfat, Tetrafluoroborat, Phosphat, Hexafluorophosphat oder Tetrachlorozinkat genannt werden.

Beispiele für die als Substituenten im Rahmen dieser Anmeldung genannten C₁-C₄-Alkylreste sind die Gruppen Methyl, Ethyl, Propyl, Isopropyl, n-Butyl, sec-Butyl und tert.-Butyl. Ethyl und Methyl sind bevorzugte Alkylreste. Beispiele für bevorzugte C₂-C₄-Alkenylreste sind Vinyl und Allyl. Erfindungsgemäß bevorzugte C₁-C₄-Alkoxyreste sind beispielsweise eine Methoxy- oder eine Ethoxygruppe. Weiterhin können als bevorzugte Beispiele für eine C₁-C₄-Hydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 1,2-Dihydroxyethylgruppe und die 2,3-Dihydroxypropylgruppe genannt werden. Eine 2-Hydroxyethylgruppe und eine 2,3-Dihydroxypropylgruppe sind besonders bevorzugt. Ein bevorzugter C₁-C₄-Perfluoralkylrest ist die Trifluormethylgruppe. Die Methoxycarbonyl-, Ethoxycarbonyl-, n-Propoxycarbonyl-, Isopropoxycarbonyl-, n-Butoxycarbonyl-, sec-Butoxycarbonyl- und tert-Butoxycarbonylgruppe sind Beispiele für C₁-C₄-Alkoxycarbonylgruppen; die Methoxycarbonyl- und die Ethoxycarbonylgruppe sind dabei besonders bevorzugt. Eine bevorzugte Hydroxy-C₁-C₄-Alkoxygruppe ist die 2-Hydroxyethoxygruppe. Bevorzugte Arylgruppen sind Phenyl, Naphthyl und Biphenyl. Beispiele für eine Heteroarylgruppe sind Pyrrolidyl, 2-Furyl, 2-Thienyl, 4-Pyridyl, 3-Pyridyl, 2-Pyridyl, Triazolyl und 1-Imidazolyl. Beispiele für eine Heterozyklus-C₁₋₄-alkylgruppe sind Pyrrolidino-(C₁₋₄)-alkyl, Piperidino-(C₁₋₄)-alkyl, Morpholino-(C₁₋₄)-alkyl, 2-Furyl-(C₁₋₄)-alkyl, 2-Thienyl-(C₁₋₄)-alkyl, 4-Pyridyl-(C₁₋₄)-alkyl, 3-Pyridyl-(C₁₋₄)-alkyl, 2-Pyridyl-(C₁₋₄)-alkyl, Triazolyl-(C₁₋₄)-alkyl und 1-Imidazolyl-(C₁₋₄)-alkyl. Beispiele für Halogenatome sind F-, Cl-, oder Br-Atome, wobei Cl-Atome ganz besonders bevorzugt sind. Bevorzugte C₁-C₄-Aminoalkylgruppen sind die Aminomethyl-, die Aminoethyl und die Aminopropylgruppe. Bevorzugte C₁-C₄-Acylgruppen sind Formyl, Acetyl, Propionyl und Butyryl. Eine bevorzugte C₁₋₄-Carboxyalkylgruppe ist die 3-Carboxypropylgruppe. Beispiele für eine Aryl-C₁-C₄-alkylgruppe sind Benzyl und 2-Phenylethyl. Besonders bevorzugte C₂-C₆-Alkenylengruppen sind Vinylen und Propylen. Eine besonders bevorzugte C₄-C₆-Alkadienylengruppe ist die 1,3-Butadien-1,4-diylgruppe. Die Gruppen 1-Carboxypropylen und 1-Carboxyethylen sind bevorzugte Carboxy-(C₁-C₄)-alkylengruppen. Unter funktionellen Carbonylderivaten werden Kondensationsprodukte aus Carbonylverbindunen und Verbindungen mit einer NH₂-Funktionalität verstanden. Beispiele für funktionelle Carbonylderivate sind Oxime und Imine. Die weiteren verwendeten Begriffe leiten sich erfindungsgemäß von den hier gegebenen Definitionen ab.

Die Verbindungen mit den Formeln I, IIa und IIb sind zum großen Teil literaturbekannt, im Handel erhältlich oder nach bekannten Syntheseverfahren herstellbar. Sie können auch in Form ihrer Hydrate vorliegen.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasem verwendet werden.

Vorzugsweise sind die Verbindungen mit der Formel I ausgewählt aus
Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron,
Salicylaldehyd, Vanillin, Coniferylaldehyd, 2,4-Dihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 4-Piperidinobenzaldehyd, 4-Dimethylaminoacetophenon, 4-Hydroxynaphthaldehyd, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 2,3,6,7-Tetrahydro-1H,5H-benzo[ij]chinolizin-9-carboxaldehyd, 4-Dimethylamino-2-methoxybenzaldehyd, 2,3,6,7-Tetrahydro-8-hydroxy-1 H,5H-benzo[ij]chinolizin-9-carboxaldehyd, 4-(1-Imidazolyl)-benzaldehyd, 2-Morpholinobenzaldehyd, Indol-3-carboxaldehyd, 1-Methylindol-3-carboxaldehyd, N-Ethylcarbazol-3-aldehyd, 2-Formylmethylen-1,3,3-trimethylindolin (Fischers Aldehyd oder Tribasen Aldehyd),
2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furtural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd,
1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon,
Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon,
4-Dimethylaminophenylpenta-2,4-dienal, 4-Diethylaminophenylpenta-2,4-dienal, 4-Methoxyphenylpenta-2,4-dienal, 3,4-Dimethoxyphenylpenta-2,4-dienal, 2,4-Dimethoxyphenylpenta-2,4-dienal, 4-Piperidinophenylpenta-2,4-dienal, 4-Morpholinophenylpenta-2,4-dienal, 4-Pyrrolidinophenylpenta-2,4-dienal,
4-Dimethylaminophenylhexa-2,4-dien-2-on, 4-Diethylaminophenylhexa-2,4-dien-2-on, 4-Methoxyphenylhexa-2,4-dien-2-on, 3,4-Dimethoxyphenylhexa-2,4-dien-2-on, 2,4-Dimethoxyphenylhexa-2,4-dien-2-on, 4-Piperidinophenylhexa-2,4-dien-2-on, 4-Morpholinophenylhexa-2,4-dien-2-on, 4-Pyrrolidinophenylhexa-2,4-dien-2-on,
4-Dimethylamino-1-naphthylpenta-2,4-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Benzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
4-Trimethylammoniobenzaldehyd-, 4-Benzyldimethylammoniobenzaldehyd-, 4-Trimethylammoniozimtaldehyd-, 4-Trimethylammonionaphthaldehyd-, 2-Methoxy-4-trimethylammoniobenzaldehyd-, 4-Acetylphenyl-trimethylammonium-, 4-(N,N-Diethylmethylammonio)-benzaldehyd-, 4-Benzoylphenyl-trimethylammonium-, N-(4-Benzoylphenyl)-N,N-diethylmethylammonium-, N-(4'-Formyl-phenyl-N-methylpyrrolidinium-, N-(4'-Formylphenyl-N-methylpiperidinium-, N-(4'-Formylphenyl-N-methylmorpholinium-, N-(4'-Acetylphenyl-N-methylmorpholinium-, N-(4'-Benzoylphenyl-N-methylmorpholinium-, 3-Formyl-N-ethyl-N-methylcarbazolium-, 1-(4'-Acetylphenyl)-3-methylimi-dazolium-, 1-(4'-Acetylphenyl)-3-methyl-2-imidazolinium-, 1-(4'-Benzoylylphenyl)-3-methylimidazolium-, 5-Acetyl-1,3-diethyl-2-methylbenzimidazolium-, 5-Trimethylammonio-1-indanon-halogenid, -benzolsulfonat, -p-toluolsulfonat, -C₁-C₄-Alkansulfonat, -C₁-C₄-alkylsulfat, - trifluormethansulfonat, -perchlorat, -sulfat, -hydrogensulfat, -tetrafluoroborat, -phosphat, -hexafluorophosphat oder -tetrachlorozinkat,
4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1 - methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolinium-, 1,3-Dimethyl-2-(4'-formylphenyl)-imidazolinium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-, 1-Methyl-2-[2-(4'-formylphenyl)-ethenyl]-pyridinium-, 1-Methyl-4-[2-(4'-acetylphenyl)-ethenyl]-pyridinium-, 1-Benzyl-4-[2-(4'-formylphenyl)-ethenyl]-pyridinium, 1-Methyl-4-[2-(4'-formylphenyl)-ethenyl]-pyridinium-, 1-Methyl-2-[2-(4-formylphenyl)-ethenyl]-pyridinium-, 1-Methyl-4-[2-(4'-formylphenyl)-ethenyl]-chinolinium, 1-Methyl-2-[2-(4'-formylphenyl)-ethenyl]-chinolinium-, 1-Methyl-2-[2-(5'-formyl-2'-furyl)-ethenyl]-chinolinium-, 1-Methyl-2-[2-(5'-formyl-2'-thienyl)-ethenyl]-chinolinium-, 1-Methyl-2-[2-(4-formylphenyl)-ethenyl]-benzothiazolinium-, 1,3-Dimethyl-2-[2-(4'-formylphenyl)-ethenyl]-benzimidazolinium-, 1,3-Dimethyl-2-[2-(4'-formylphenyl)-ethenyl]-imidazolinium-, 1-Methyl-5-oxo-indeno[1,2-b]pyridinium(-4-methyl-4-azonio-9-fluorenon-), 1-Ethyl-5-oxo-indeno[1,2-b]pyridinium(-4-Ethyl-4-azonio-9-fluorenon-), 1-Benzyl-5-oxo-indeno[1,2-b]pyridinium(-4-benzyl-4-azonio-9-fluorenon-), 2-Methyl-5-oxo-indeno[1,2-c]pyridinium-, 2-Methyl-9-oxo-indeno[2,1-c]pyridinium-, 1-Methyl-9-oxo-indeno[2,1-b]pyridinium-benzolsulfonat, -p-toluolsulfonat, -methansulfonat, -perchlorat, - sulfat, -chlorid, -bromid, -iodid, -tetrachlorozinkat, -methylsulfat-, trifluormethansulfonat, - tetrafluoroborat,
Isatin, 1-Methyl-isatin, 1-Allyl-isatin, 1-Hydroxymethyl-isatin, 5-Chlor-isatin, 5-Methoxyisatin, 5-Nitroisatin, 6-Nitro-isatin, 5-Sulfo-isatin, 5-Carboxy-isatin, Chinisatin, 1-Methylchinisatin,
Acenaphthenchinon, Benzil, 2,2'-Furil, 2,2'-Pyridil, 4,4'-Pyridil, Salicil, Anisil,
sowie beliebigen Gemischen der voranstehenden.

Vorzugsweise sind die Verbindungen mit der Formel IIa bzw. IIb ausgewählt aus 4-Amino-2,3-dimethyl-1-phenyl-3-pyrazolin-5-on (4-Aminoantipyrin), 4-Amino-2,3-dimethyl-1-(4'-chlorphenyl)-3-pyrazolin-5-on, 4-Amino-2,3-dimethyl-1-(2'-chlorphenyl)-3-pyrazolin-5-on, 4-Amno-2,3-dimethyl-1-(4'-fluorphenyl)-3-pyrazolin-5-on, 4-Amino-2,3-dimethyl-1-(4'-methoxyphenyl)-3-pyrazolin-5-on, 4-Amino-2,3-dimethyl-1-(p-tolyl)-3-pyrazolin-5-on, 4-Amino-2-ethyl-3-methyl-1-phenyl-3-pyrazolin-5-on, 4-Amino-2,3-dimethyl-1-(2',4'-dichlorphenyl)-3-pyrazolin-5-on, 4-Amino-1,2-diphenyl-3-hydroxy-3-pyrazolin-5-on, 4-Amino-1,2-bis-(4'-chlorphenyl)-3-hydroxy-3-pyrazolin-5-on, 4-Amino-3-methyl-1-phenyl-2-pyrazolin-5-on, 4-Amino-3-methyl-1-(4'-sulfophenyl)-2-pyrazolin-5-on, 4-Amino-3-methyl-1-(4'-methoxyphenyl)-2-pyrazolin-5-on, 4-Amino-3-methyl-1-(4'-chlorphenyl)-2-pyrazolin-5-on, 4-Amino-3-methyl-1-(3'-chlorphenyl)-2-pyrazolin-5-on, 4-Amino-3-carbamoyl-1-phenyl-2-pyrazolin-5-on, 4-Amino-3-carboxy-1-phenyl-2-pyrazolin-5-on, 4-Amino-3-ethoxycarboyl-1-phenyl-2-pyrazolin-5-on, 4-Amino-1-phenyl-2-pyrazolin-5-on, 4-Amino-1-(4'-chlorphenyl)-2-pyrazolin-5-on, 4-Amino-1-(4'-fluorphenyl)-2-pyrazolin-5-on, 4-Amino-1-(4'-chlorphenyl)-2-pyrazolin-5-on, 4-Amino-1-(4'-methoxyphenyl)-2-pyrazolin-5-on, 4-Amino-1-(4'-carboxyphenyl)-2-pyrazolin-5-on sowie ihre hautverträglichen Säureadditionsprodukte.

Die voranstehend genannten Verbindungen mit der Formel I, IIa oder IIb werden vorzugsweise in den erfindungsgemäßen Mitteln jeweils in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von üblichen Oxidationsfarbstoffvorprodukten eingesetzt werden.

In allen Färbemitteln können auch mehrere verschiedene Verbindungen mit der Formel I und der Formel IIa bzw. IIb gemeinsam zum Einsatz kommen.

Unter die voranstehend beschriebene Ausführungsform fällt auch die Verwendung von solchen Substanzen, die Reaktionsprodukte von aromatischen oder heteroaromatischen Aldehyden und/oder Ketonen gemäß Formel I und 4-Aminopyrazolin-5-onen mit der Formel IIa bzw. IIb als direktziehende Färbemittel. Derartige Reaktionsprodukte können z. B. durch kurzes Erwärmen der beiden Komponenten in stöchiometrischen Mengen in wässrigem neutralen bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

Färbemittel, die als färbende Komponente die erfindungsgemäße Kombination enthalten, werden bevorzugt für Färbungen im Nuancenbereich von gelb über gelbbraun, orange, braunorange, rot bis hin zu rotbraun eingesetzt. Färbungen mit noch erhöhter Brillanz und verbesserten Echtheitseigenschaften (Lichtechtheit, Waschechtheit, Reibechtheit) können erzielt werden, wenn die erfindungsgemäß eingesetzte Kombination aus den Verbindungen mit den Formel I, IIa und/oder IIb gemeinsam mit mindestens einer weiteren Komponente (im folgenden Komponente B genannt), ausgewählt aus Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren, aus 2 bis 9 Aminosäuren aufgebauten Oligopeptiden und deren physiologisch verträglichen Salzen, sowie CH-aciden Verbindungen und quartären Ammoniumverbindungen verwendet werden. Dies sind einerseits Verbindungen, die für sich alleine keratinhaltige Fasern nur schwach färben und erst gemeinsam mit den Verbindungen der Formel I brillante Färbungen ergeben. Andererseits sind darunter aber auch Verbindungen, die bereits als Oxidationsfarbstoffvorprodukte eingesetzt werden.

Geeignete Verbindungen mit primärer oder sekundärer Aminogruppe als Komponente B sind z. B. primäre aromatische Amine wie N,N-Dimethyl-, N,N-Diethyl-, N-(2'-Hydroxyethyl)-N-ethyl-, N,N-Bis-(2'-hydroxyethyl)-, N-(2'-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, 2-Aminomethyl-4-aminophenol, 2-Hydroxymethyl-4-aminophenol, o-, p-Phenylendiamin, o-Toluylendiamin, 2,5-Diaminotoluol, -phenol, - phenethol, 4-Amino-3-methylphenol, 2-(2',5'-Diaminophenyl)-ethanol, 2,4-Diaminophenoxyethanol, 2-(2',5'-Diaminophenoxy)-ethanol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2'-hydroxyethylamino)-, 6-Methyl-3-amino-2-chlor-, 2-Methyl-5-amino-4-chlor-, 3,4-Methylendioxy-, 5-(2'-Hydroxyethylamino)-4-methoxy-2-methyl-, 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, 1,4-Bis-(4'-aminophenyl)-1,4-diazacycloheptan, Bis-(5'-amino-2'-hydroxyphenyl)-methan, aromatische Nitrile, Nitrogruppen-haltige Aminoverbindungen, wie 3-Amino-6-methylamino-2-nitro-pyridin, Pikraminsäure, [8-[(4'-Amino-2'-nitrophenyl)azo]-7-hydroxynaphth-2-yl]-trimethylammoniumchlorid, [8-((4'-Amino-3'-nitrophenyl)azo)-7-hydroxynaphth-2-yl]-trimethylammoniumchlorid (Basic Brown 17), 1-Hydroxy-2-amino-4,6-dinitrobenzol, 1-Amino-2-nitro-4-bis-(2'-hydroxyethyl)-aminobenzol, 1-Amino-2-(2'-hydroxyethyl)-amino-5-nitrobenzol (HC Yellow Nr. 5), 1-Amino-2-nitro-4-(2'-hydroxyethyl)-aminobenzol (HC Red Nr. 7), 2-Chloro-5-nitro-N-hydroxyethyl-1,4-phenylendiamin, 1-(2'-Hydroxyethyl)-amino-2-nitro-4-aminobenzol (HC Red Nr. 3), 4-Amino-3-nitrophenol, 4-Amino-2-nitrophenol, 6-Nitro-o-toluidin, 1-Amino-3-methyl-4-(2'-hydroxyethyl)-amino-6-nitrobenzol (HC Violet Nr. 1), 1-Amino-2-nitro-4-(2',3'-dihydroxypropyl)-amino-5-chlorobenzol (HC Red Nr. 10), 2-(4'-Amino-2'-nitroanilino)-benzoesäure, 6-Nitro-2,5-diaminopyridin, 2-Amino-6-chloro-4-nitrophenol, 1-Amino-2-(3'-nitrophenylazo)-7-phenylazo-8-naphthol-3,6-disulfonsäure Dinatriumsalz (Acid blue Nr. 29), 1-Amino-2-(2'-hydroxy-4'-nitrophenylazo)-8-naphthol-3,6-disutfonsäure Dinatriumsalz (Palatinchrome green), 1-Amino-2-(3'-chlor-2'-hydroxy-5'-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2'-nitrobenzylidenamino)-benzol, 2-[2'-(Diethylamino)-ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2'-hydroxyethylamino)-benzol, 2-Nitro-1-amino-4-[bis-(2'-hydroxyethyl)-amino]-benzol, 1-Amino-5-chlor-4-(2'-hydroyethylamino)-2-nitrobenzol, aromatische Aniline bzw. Phenole mit einem weiteren aromatischen Rest, wie sie in der Formel III dargestellt sind 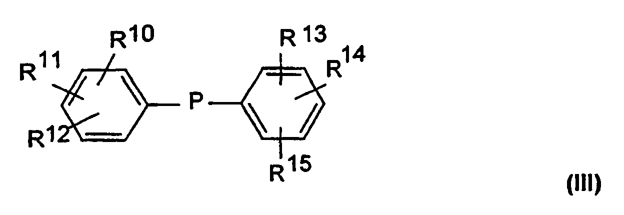 in der
- R¹² für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
- R¹³, R¹⁴, R¹⁵, R¹⁶ und R¹⁷ unabhängig voneinander für ein Wasserstoffatom, eine Hydroxy- oder eine Aminogruppe, die durch C₁₋₄-Alkyl-, C₁₋₄-Hydroxyalkyl, C₁₋₄-Alkoxy-, C₁₋₄-Aminoalkyl- oder C₁₋₄-Alkoxy-C₁₋₄-alkylgruppen substituiert sein kann, stehen, und
- P für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel IV

   **Q'-(CH**_{**2**}**-Q-CH**_{**2**}**-Q")**_{**o**} **(IV)**
in der
- Q eine direkte Bindung, eine CH₂- oder CHOH-Gruppe bedeutet,
- Q' und Q" unabhängig voneinander für ein Sauerstoffatom, eine NR¹⁸-Gruppe, worin R¹⁸ ein Wasserstoffatom, eine C₁₋₄-Alkyl- oder eine Hydroxy-C₁₋₄-alkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ'-O, worin p und p' 2 oder 3 sind, stehen und
- o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraaminobenzophenon, 1,3-Bis-(2',4'-diaminophenoxy)-propan, 1,8-Bis-(2',5'-diaminophenoxy)-3,6-dioxaoctan, 1,3-Bis-(4'-aminophenylamino)propan, -2-propanol, 1,3-Bis-[N-(4'-aminophenyl)-2-hydroxyethylamino]-2-propanol, N,N-Bis-[2-(4'-aminophenoxy)-ethyl]-methylamin, N-Phenyl-1,4-phenylendiamin, 1,4-Bis-(4'-aminophenyl)-1,4-diazacycloheptan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 1-Phenyl-, 1-(2'-Hydroxyethyl)-4,5-diaminopyrazol, 1-Phenyl-3-methyl-4,5-diaminopyrazol, 2-, 3-, 8-Aminochinolin, 4-Aminochinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indol- und Indolinderivaten, wie 4-, 5-, 6-, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2'-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als Aminosäuren kommen bevorzugt alle natürlich vorkommenden und synthetischen α-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Omithin, Prolin, Lysin und Tryptophan. Aber auch andere Aminosäuren, wie z.B. 6-Aminocapronsäure und β-Alanin, können eingesetzt werden.

Die Oligopeptide können dabei natürlich vorkommende oder synthetische Oligopeptide, aber auch die in Polypeptid- oder Proteinhydrolysaten enthaltenen Oligopeptide sein, sofem sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Als CH-acide Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 1,2-Dimethyl-naphtho[1 ,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-Ethyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Oxindol, 3-Indoxylacetat, Cumaranon, 1-Methyl-3-phenyl-2-pyrazolinon, Indan-1,3-dion, Indan-1-on, 1-Dicyanmethylenindan und 1,3-Bis-(dicyanmethylen)-indan.

Beispiele für quartäre Ammoniumsalze sind Tetramethyl-, Tetraethyl-, Tetrapropyl-, Tetrabutyl-, Benzyltrimethylammonium-chlorid, -bromid, -iodid, -hydrogensulfat, - (halb)sulfat sowie Polyquaternium 10.

Die Verbindungen der Komponente B werden besonders bevorzugt ausgewählt aus der Gruppe bestehend aus N-(2'-Hydroxyethyl)-N-ethyl-, 2-Chlor-p-phenylendiamin, N,N-Bis-(2'-hydroxyethyl)-p-phenylendiamin, 4-Aminophenol, 2-Amino-6-chloro-4-nitrophenol, p-Phenylendiamin, 2-(2',5'-Diaminophenyl)-ethanol, 2,5-Diaminotoluol, 3,4-Methylendioxyanilin, 2-Amino-4-(2'-hydroxyethylamino)-anisol, 2-(2',4'-Diaminophenoxy)-ethanol, 3-Amino-2,4-dichlor-, 2-Methyl-5-amino-, 3-Methyl-4-amino-, 2-Methyl-5-(2'-hydroxyethylamino)-, 2-Methyl-5-amino-4-chlor-, 6-Methyl-3-amino-2-chlor-, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triami-no-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 4,5-Diamino-1-(2'-hydroxyethyl)-pyrazol, 5,6-Dihydroxyindol und 5,6-Dihydroxyindolin, Tetrabutylammoniumbromid, β-Alanin, L-Prolin, L-Lysin, DL-Tyrosin sowie deren mit vorzugsweise anorganischen Säuren gebildeten physiologisch verträglichen Salze.

Die voranstehend genannten Verbindungen der Komponente B können in einer Menge von jeweils 0,03 bis 65 mmol, insbesondere 1 bis 40 mmol, jeweils bezogen auf 100 g des gesamten Färbemittels, eingesetzt werden. Weiterhin kann es erfindungsgemäß besonders bevorzugt sein, die Verbindungen mit den Formeln I, IIa und IIb sowie die Verbindung der Komponente B im molaren Mengenverhältnis von 4 : 1 : 1 bis 1 : 2 : 2, insbesondere etwa 2 : 1 : 1, einzusetzen.

In einer weiteren Ausführungsform kann die Bandbreite des Nuancenbereichs der Ausfärbungen durch die Verwendung von reaktiven Carbonylverbindungen als zusätzliche Komponente C erweitert werden. Beispiele für geeignete reaktive Carbonylverbindungen sind Aldehyde oder cyclische oder nichtcyclische Ketone. Diese Verbindungen können mit den Aminoverbindungen bzw. CH-aciden Verbindungen der Komponente B reagieren. Durch geeignete Kombination der Verbindungen mit der Formel I, der Komponente B und ggf. C läßt sich eine große Vielfalt an Ausfärbungen erhalten. Unter diese Ausführungsform fällt auch die Verwendung von solchen Substanzen, die Reaktionsprodukte von Nitroxanthenderivaten der Formel I mit den genannten Verbindungen der Komponente B und Verbindungen der Komponente C darstellen, als direktziehende Färbemittel.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65, insbesondere 1 bis 40 mmol, jeweils bezogen auf 100 g des gesamten Färbemittels, eingesetzt werden.

Auf die Anwesenheit von Oxidationsmitteln, z. B. H₂O₂, kann dabei verzichtet werden. Es kann jedoch unter Umständen wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red BN, Basic Red 76, HC Blue 2, Disperse Blue 3, Basic Blue 99, Disperse Violet 1, Disperse Violet 4, Disperse Black 9, Basic Brown 16 und Basic Brown 17 bekannten Verbindungen sowie 6-Nitro-1,2,3,4-tetrahydrochinoxalin, 4-N-Ethyl-1,4-bis-(2'-hydroxyethylamino)-2-nitrobenzolhydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein weiterer Gegenstand der vorliegenden Erfindung betrifft die Verwendung einer Kombination aus
aromatischen oder heteroaromatischen Aldehyden und/oder Ketonen der Formel I oder physiologisch verträglichen Salzen davon, 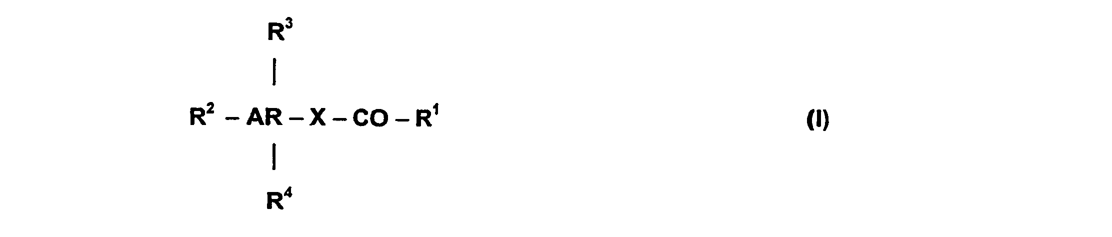 in der AR, R¹, R², R³, R⁴ und X wie oben definiert sind,
und
mindestens einem 4-Aminopyrazolin-5-on mit der Formel (IIa) oder (IIb) oder deren physiologisch verträgliche Salze, 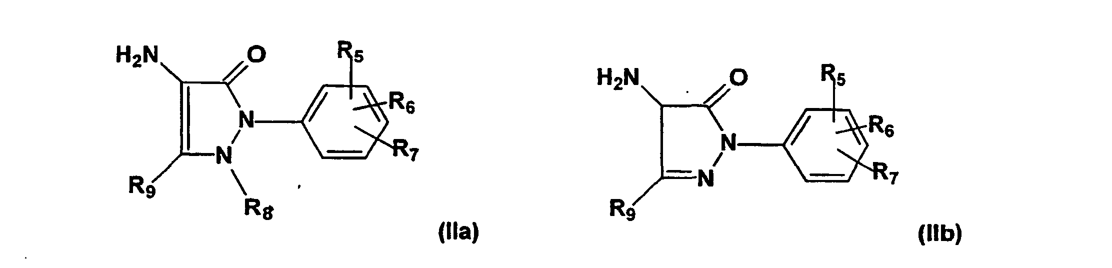 in denen R⁵, R⁶, R⁷, R⁸ und R⁹ wie oben definiert sind,
als färbende Komponenten in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend
A1) mindestens ein aromatisches oder heteroaromatisches Aldehyd und/oder Keton mit der Formel I oder physiologisch verträgliche Salze davon, 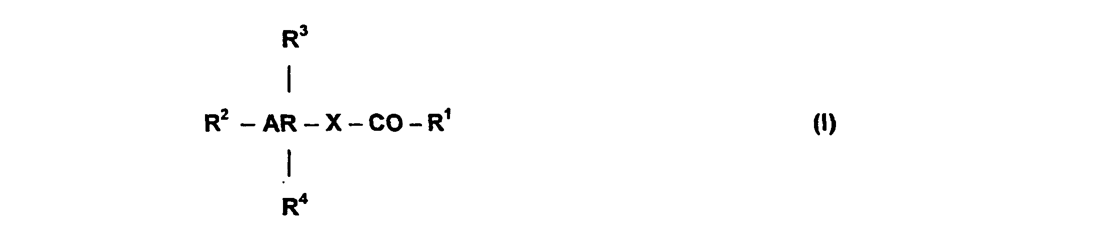 in der AR, R¹, R², R³, R⁴ und X wie oben definiert sind, und
A2) mindestens ein 4-Aminopyrazolin-5-on mit der Formel (IIa) oder (IIb) oder deren physiologisch verträgliche Salze, 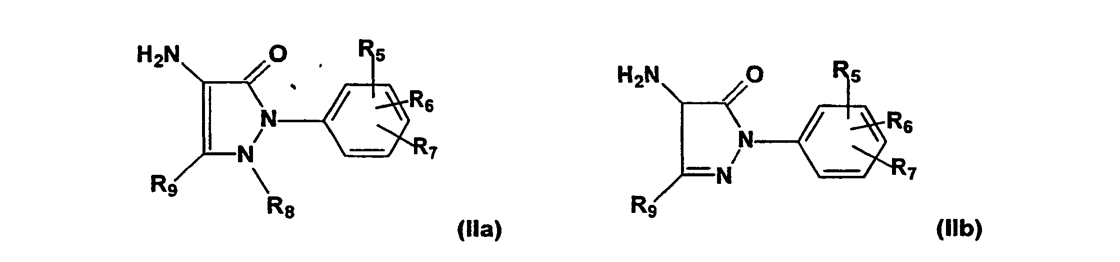 in der R⁶, R⁶, R⁷, R⁸ und R⁹ wie oben definiert sind, und
B) gegebenenfalls mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, aromatischen Hydroxyverbindungen, Aminosäuren, aus 2 bis 9 Aminosäuren aufgebauten Oligopeptiden und/oder mindestens eine CH-acide Verbindung oder quartäre Ammoniumverbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Kombination aromatischen oder heteroaromatischen Aldehyden und/oder Ketonen der Formel I und 4-Aminopyrazolin-5-onen der Formel IIa bzw. IIb und die Verbindungen der Komponente B können entweder gleichzeitig auf das Haar aufgebracht werden oder aber auch nacheinander, wobei es unerheblich ist, welche der Komponenten zuerst aufgetragen wird. Die fakultativ enthaltenen Ammonium- oder Metallsalze können dabei den Verbindungen mit der Formel I, IIa oder IIb oder den Verbindungen der Komponente B zugesetzt werden. Zwischen dem Auftragen der einzelnen Komponenten können bis zu 30 Minuten Zeitabstand liegen. Auch eine Vorbehandlung der Fasern mit der Salzlösung ist möglich.

Die aromatischen oder heteroaromatischen Aldehyde und/oder Ketone mit der Formel I, die 4-Aminopyrazolin-5-one mit der Formel IIa bzw. IIb und die Verbindungen der Komponente B können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellung einer Färbelösung

Es wurde je eine Aufschlämmung von 5 mmol des Aldehyd oder Ketons der Formel I, 5 mmol des 4-Aminopyrazolin-5-ons der Formel II, 5 mmol Natriumacetat, 5 mmol Piperidin und einen Tropfen einer 20%igen Fettalkylethersulfat-Lösung in 50 ml Wasser bei 50°C hergestellt. Die Aufschlämmungen wurden nach Abkühlen auf 30°C miteinander vermischt und mit verdünnter NaOH oder Salzsäure auf einen pH-Wert von 6 eingestellt.

In diese Färbemischung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft (30 bis 40°C) getrocknet und anschließend ausgekämmt.

Die jeweiligen Farbnuancen und Farbtiefen sind in der nachfolgenden Tabelle 1 wiedergegeben.

Die Farbtiefe wurde dabei nach folgender Skala bewertet:

| | |
|---|---|
| - | keine oder eine sehr blasse Ausfärbung |
| (+) | schwache Intensität |
| + | mittlere Intensität |
| +(+) | mittlere bis starke Intensität |
| ++ | starke Intensität |
| ++(+) | starke bis sehr starke Intensität |
| +++ | sehr starke Intensität |

**Tabelle 1**

| **Ausfärbungen mit einer Kombination aus aromatischen oder heteroaromatische** **Aldehyden und/oder Ketonen und 4-Aminopyrazolin-5-onen** | | | |
|---|---|---|---|
| **Aldehyd oder Keton der Formel I** | **4-Aminopyrazolin-5-on der Formel II** | **Farbe** | **Int.** |
| 4-Formyl-1-methylpyridiniumbenzosulfonat | 4-Aminoantipyrin | goldgelb | ++(+) |
| 4-Formyl-1-methylchinolinium-p-toluolsulfonat- | 4-Aminoantipyrin | orangerot | ++(+) |
| Coniferylaldehyd | 4-Aminoantipyrin | hellorangegelb | + |
| 4-Dimethylaminobenzaldehyd | 4-Aminoantipyrin | hellgelb | + |
| Isatin | 4-Aminoantipyrin | orange | ++(+) |

## Patentansprüche

1. Mittel zum Färben von keratinhaltigen Fasern, insbesondere menschlichen Haaren, enthaltend mindestens ein aromatisches oder heteroaromatisches Aldehyd und/oder Keton mit der Formel I oder physiologisch verträgliche Salze davon, 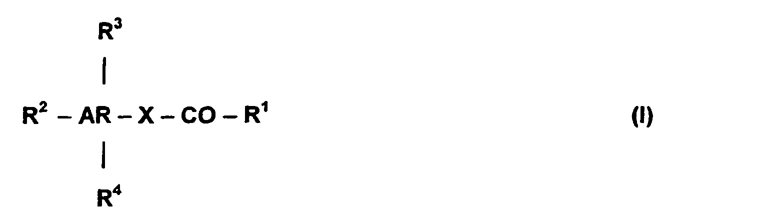 wobei
• AR steht für Benzol, Naphthalin, Pyridin, Pyrimidin, Pyrazin, Pyrazidin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
• R¹ steht für ein Wasserstoffatom, eine C₁-C₄-Alkyl-, C₁-C₅-Acyl-, C₂-C₄-Alkenyl-, C₁-C₄-Perfluoralkyl-, eine ggf. substituierte Aryl- oder Heteroarylgruppe,
• R², R³ und R⁴ stehen unabhängig voneinander für ein Wasserstoffatom, ein Halogenatom, eine C₁-C₄-Alkyl-, C₁₋₄-Alkoxy-, C₁-C₄-Aminoalkyl-, C₁-C₄-Hydroxyalkylgruppe, eine C₁-C₄-Acylgruppe, eine Carboxy-, Carboxylato-, Sulfo-, Sulfato-, C₂-C₆-Alkenyl-, Aryl-, eine Aryl-C₁-C₄-alkylgruppe, eine Hydroxy-, Nitro-, Pyrrolidino-, Morpholino-, Piperidino-, Amino- bzw. Ammonio- oder 1-Imidazol(in)iogruppe, wobei die letzten drei Gruppen mit C₁₋₄-Alkyl-, C₁₋₄-Carboxyalkyl-, C₁₋₄-Hydroxyalkyl-, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können, durch C₁-C₄-Alkyl-, C₂-C₆-Alkenyl- oder C₁-C₄-Hydroxyalkoxygruppen substituiert sein können, wobei auch zwei der Reste aus R², R³, R⁴ und -X-CO-R¹ einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R², R³ und R⁴,
• X steht für eine direkte Bindung, eine Carbonyl-, eine Carboxy-(C₁-C₄)-alkylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei X zusammen mit der CO-R¹-Gruppe auch einen gegebenenfalls substituierten 5-, 6- oder 7-Ring bilden kann,
sowie mindestens ein 4-Aminopyrazolin-5-on mit der Formel (IIa) oder (IIb) oder deren physiologisch verträgliche Salze, 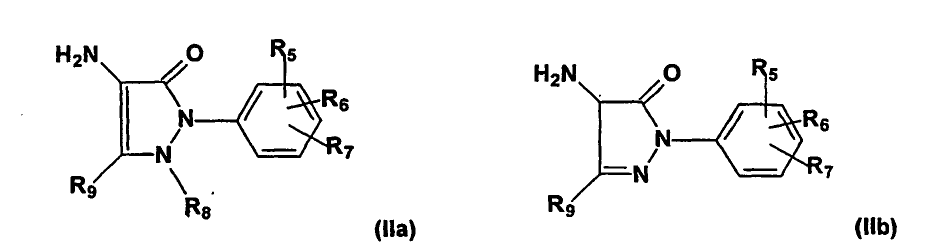 in denen
• R⁵, R⁶ und R⁷ stehen unabhängig voneinander für ein Wasserstoffatom, ein Halogenatom, eine C₁₋₄-Alkyl-, C₁₋₄-Alkoxy- oder C₁₋₄-Hydroxyalkoxygruppe, eine Hydroxy-, Carboxy-, Carboxylato-, C₁₋₄-Alkoxycarbonyl-, gegebenenfalls substituierte Carbamoyl-, Sulfo-, Sulfato-, Sulfamoylgruppe oder Aminogruppe, die durch C₁₋₄-Alkyl- oder Hydroxyalkylgruppen substituiert sein kann,
• R⁸ und R⁹ stehen unabhängig voneinander für ein Wasserstoffatom, eine Hydroxy-, Amino-, C₁₋₄-Alkyl-, C₁₋₄-Alkoxy-, Carbamoyl-, Carboxyl-, C₁₋₄-Alkoxycarbonyl-, Aryl-, Aryl-C₁₋₄-alkyl- oder Heteroarylgruppe.

2. Mittel nach Anspruch 1, **dadurch gekennzeichnet, dass** als Verbindungen mit der Formel I
Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron,
Salicylaldehyd, Vanillin, Coniferylaldehyd, 2,4-Dihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 4-Piperidinobenzaldehyd, 4-Dimethylaminoacetophenon, 4-Hydroxynaphthaldehyd, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylaminobenzaldehyd, 4-Diphenylaminobenzaldehyd, 2,3,6,7-Tetrahydro-1H,5H-benzo[ij]chinolizin-9-carboxaldehyd, 4-Dimethylamino-2-methoxybenzaldehyd, 2,3,6,7-Tetrahydro-8-hydroxy-1H,5H-benzo[ij]chinolizin-9-carboxaldehyd, 4-(1-Imidazolyl)-benzaldehyd, 2-Morpholinobenzaldehyd, Indol-3-carboxaldehyd, 1-Methylindol-3-carboxaldehyd, N-Ethylcarbazol-3-aldehyd, 2-Formylmethylen-1,3,3-trimethylindolin (Fischers Aldehyd oder Tribasen Aldehyd),
2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2'-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd,
1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon,
Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 3-Benzyliden-1,3-cyclohexandion, 3-(4'-Hydroxybenzyliden)-1,3-cyclohexandion, 3-(4'-Dimethylaminobenzyliden)-1,3-cyclohexandion, 3-Benzyliden-5,5-dimethyl-1,3-cyclohexandion, 3-(4'-Hydroxybenzyliden)-5,5-dimethyl-1,3-cyclohexandion, 3-(4'-Hydroxy-3-methoxybenzyliden)-5,5-dimethyl-1,3-cyclohexandion, 3-(4'-Dimethylaminobenzyliden)-5,5-dimethyl-1,3-cyclohexandion, 2-Benzylidencyclopentanon, 2-(4'-Hydroxybenzyliden)-cyclopentanon, 2-(4'-Dimethylaminobenzyliden)-cyclopentanon,
4-Dimethylaminophenylpenta-2,4-dienal, 4-Diethylaminophenylpenta-2,4-dienal, 4-Methoxyphenylpenta-2,4-dienal, 3,4-Dimethoxyphenylpenta-2,4-dienal, 2,4-Dimethoxyphenylpenta-2,4-dienal, 4-Piperidinophenylpenta-2,4-dienal, 4-Morpholinophenylpenta-2,4-dienal, 4-Pyrrolidinophenylpenta-2,4-dienal,
4-Dimethylaminophenylhexa-2,4-dien-2-on, 4-Diethylaminophenylhexa-2,4-dien-2-on, 4-Methoxyphenylhexa-2,4-dien-2-on, 3,4-Dimethoxyphenylhexa-2,4-dien-2-on, 2,4-Dimethoxyphenylhexa-2,4-dien-2-on, 4-Piperidinophenylhexa-2,4-dien-2-on, 4-Morpholinophenylhexa-2,4-dien-2-on, 4-Pyrrolidinophenylhexa-2,4-dien-2-on, 4-Dimethylamino-1-naphthylpenta-2,4-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Benzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
4-Trimethylammoniobenzaldehyd-, 4-Benzyldimethylammoniobenzaldehyd-, 4-Trimethylammoniozimtaldehyd-, 4-Trimethylammonionaphthaldehyd-, 2-Methoxy-4-trimethylammoniobenzaldehyd-, 4-Acetylphenyl-trimethylammonium-, 4-(N,N-Diethyl-methylammonio)-benzaldehyd-, 4-Benzoylphenyl-trimethylammonium-, N-(4'-Benzoyl-phenyl)-N,N-diethylmethylammonium-, N-(4'-Formyl-phenyl-N-methylpyrrolidinium-, N-(4'-Formylphenyl-N-methylpiperidinium-, N-(4'-Formylphenyl-N-methylmorpholinium-, N-(4'-Acetylphenyl-N-methylmorpholinium-, N-(4'-Benzoylphenyl-N-methylmorpholinium-, 3-Formyl-N-ethyl-N-methylcarbazolium-, 1-(4'-Acetylphenyl)-3-methylimidazolium-, 1-(4'-Acetylphenyl)-3-methyl-2-imidazolinium-, 1-(4'-Benzoylylphenyl)-3-methylimidazolium-, 5-Acetyl-1,3-diethyl-2-methylbenzimidazolium-, 5-Trimethylammonio-1-indanon-halogenid, - benzolsulfonat, -p-toluolsulfonat, -C₁-C₄-Alkansulfonat, -C₁-C₄-alkylsulfat, - trifluormethansulfonat, -perchlorat, -sulfat, -hydrogensulfat, -tetrafluoroborat, -phosphat, -hexafluorophosphat oder -tetrachlorozinkat,
4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolinium-, 1,3-Dimethyl-2-(4'-formylphenyl)-imidazolinium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-, 1-Methyl-2-[2-(4'-formylphenyl)-ethenyl]-pyridinium-, 1-Methyl-4-[2-(4'-acetylphenyl)-ethenyl]-pyridinium-, 1-Benzyl-4-[2-(4'-formylphenyl)-ethenyl]-pyridinium, 1-Methyl-4-[2-(4'-formylphenyl)-ethenyl]-pyridinium-, 1-Methyl-2-[2-(4'-formylphenyl)-ethenyl]-pyridinium-, 1-Methyl-4-[2-(4'-formylphenyl)-ethenyl]-chinolinium, 1-Methyl-2-[2-(4'-formylphenyl)-ethenyl]-chinolinium-, 1-Methyl-2-[2-(5'-formyl-2'-furyl)-ethenyl]-chinolinium-, 1-Methyl-2-[2-(5'-formyl-2'-thienyl)-ethenyl]-chinolinium-, 1-Methyl-2-[2-(4'-formylphenyl)-ethenyl]-benzothiazolinium-, 1,3-Dimethyl-2-[2-(4'-formylphenyl)-ethenyl]-benzimidazolinium-, 1,3-Dimethyl-2-[2-(4'-formylphenyl)-ethenyl]-imidazolinium-, 1-Methyl-5-oxo-indeno[1,2-b]pyridinium- (4-Methyl-4-azonio-9-fluorenon-), 1-Ethyl-5-oxo-indeno[1,2-b]pyridinium- (4-Ethyl-4-azonio-9-fluorenon-), 1-Benzyl-5-oxo-indeno[1,2-b]pyridinium- (4-Benzyl-4-azonio-9-fluorenon-), 2-Methyl-5-oxo-indeno[1,2-c]pyridinium-, 2-Methyl-9-oxo-indeno[2,1-c]pyridinium-, 1-Methyl-9-oxo-indeno[2,1-b]pyridinium-benzolsulfonat, -p-toluolsulfonat, -methansulfonat, - perchlorat, -sulfat, -chlorid, -bromid, -iodid, -tetrachlorozinkat, -methylsulfat-, trifluormethansulfonat, -tetrafluoroborat,
Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin,
Acenaphthenchinon, Benzil, 2,2'-Furil, 2,2'-Pyridil, 4,4'-Pyridil, Salicil, Anisil,
sowie beliebige Gemische der voranstehenden eingesetzt werden.

3. Mittel nach Anspruch 1 oder 2, **dadurch gekennzeichnet, dass** als Verbindungen mit der Formel IIa bzw. IIb
4-Amino-2,3-dimethyl-1-phenyl-3-pyrazolin-5-on (4-Aminoantipyrin), 4-Amino-2,3-dimethyl-1-(4'-chlorphenyl)-3-pyrazolin-5-on, 4-Amino-2,3-dimethyl-1-(2'-chlorphenyl)-3-pyrazolin-5-on, 4-Amino-2,3-dimethyl-1-(4'-fluorphenyl)-3-pyrazolin-5-on, 4-Amino-2,3-dimethyl-1-(4'-methoxyphenyl)-3-pyrazolin-5-on, 4-Amino-2,3-dimethyl-1-(p-tolyl)-3-pyrazolin-5-on, 4-Amino-2-ethyl-3-methyl-1-phenyl-3-pyrazolin-5-on, 4-Amino-2,3-dimethyl-1-(2',4'-dichlorphenyl)-3-pyrazolin-5-on, 4-Amino-1,2-diphenyl-3-hydroxy-3-pyrazolin-5-on, 4-Amino-1,2-bis-(4'-chlorphenyl)-3-hydroxy-3-pyrazolin-5-on, 4-Amino-3-methyl-1-phenyl-2-pyrazolin-5-on, 4-Amino-3-methyl-1-(4'-sulfophenyl)-2-pyrazolin-5-on, 4-Amino-3-methyl-1-(4'-methoxyphenyl)-2-pyrazolin-5-on, 4-Amino-3-methyl-1-(4'-chlorphenyl)-2-pyrazolin-5-on, 4-Amino-3-methyl-1-(3'-chlorphenyl)-2-pyrazolin-5-on, 4-Amino-3-carbamoyl-1-phenyl-2-pyrazolin-5-on, 4-Amino-3-carboxy-1-phenyl-2-pyrazolin-5-on, 4-Amino-3-ethoxycarboyl-1-phenyl-2-pyrazolin-5-on, 4-Amino-1-phenyl-2-pyrazolin-5-on, 4-Amino-1-(4'-chlorphenyl)-2-pyrazolin-5-on, 4-Amino-1-(4'-fluorphenyl)-2-pyrazolin-5-on, 4-Amino-1-(4'-chlorphenyl)-2-pyrazolin-5-on, 4-Amino-1-(4'-methoxyphenyl)-2-pyrazolin-5-on, 4-Amino-1-(4'-carboxyphenyl)-2-pyrazolin-5-on sowie ihre hautverträglichen Säureadditionsprodukte eingesetzt werden.

4. Mittel nach einem der Ansprüche 1 bis 3, **dadurch gekennzeichnet, dass** die aromatischen oder heteroaromatischen Aldehyde und/oder Ketone der Formel I und die 4-Aminopyrazolin-5-one der Formel IIa bzw. IIb jeweils in einer Menge von 0,03 bis 65 mmol, insbesondere von 1 bis 40 mmol, bezogen auf 100 g des gesamten Färbemittels, enthalten sind.

5. Mittel nach einem der Ansprüche 1 bis 4, **dadurch gekennzeichnet, dass** es zusätzlich mindestens eine Verbindung mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren, aus 2 bis 9 Aminosäuren aufgebauten Oligopeptiden und/oder mindestens eine CH-acide Verbindung oder und quartäre Ammoniumverbindung enthält.

6. Mittel nach Anspruch 5, **dadurch gekennzeichnet, dass** die weitere Verbindung ausgewählt ist aus
primären oder sekundären Aminen aus der Gruppe, bestehend aus N-(2'-Hydroxyethyl)-N-ethyl-, N-(2'-Methoxyethyl-), 2,3-, 2,4-, 2,5-Dichlor-p-phenylendiamin, 2-Chlor-p-phenylendiamin, N,N-Bis-(2'-hydroxyethyl)-p-phenylendiamin, 2,5-Dihydroxy-4-morpholinoanilin-dihydrobromid, 2-, 3-, 4-Aminophenol, o-, m-, p-Phenylendiamin, 2,4-Diaminophenoxyethanol, 2-(2',5'-Diaminophenyl)-ethanol, 2,5-Diaminotoluol, -phenol, -phenethol, 4-Methylamino-, 3-Amino-4-(2'-hydroxyethyloxy), 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2'-hydroxyethylamino)-, 2-Methyl-5-amino-4-chlor-, 6-Methyl-3-amino-2-chlor-, 5-(2'-Hydroxyethylamino)-4-methoxy-2-methyl-, 4-Amino-2-aminomethyl-phenol, 2-Hydroxymethyl-4-aminophenol, 2-Diethylaminomethyl-4-aminophenol, 2-Dimethylaminomethyl-4-aminophenol, 2,6-Dichlor-4-aminophenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogalloldihydrochlorid, 3,5-Diamino-4-hydroxybrenzcatechin-sulfat, aromatische Nitrile, Nitrogruppen-haltige Aminoverbindungen, wie 3-Amino-6-methylamino-2-nitropyridin, Pikraminsäure, [8-[(4'-Amino-2'-nitrophenyl)azol-7-hydroxy-naphth-2-yl]-trimethylammoniumchlorid, [8-((4'-Amino-3'-nitrophenyl)azo)-7-hydroxy-naphth-2-yl]-trimethylammoniumchlorid (Basic Brown 17), 1-Hydroxy-2-amino-4,6-dinitrobenzol, 1-Amino-2-nitro-4-bis-(2'-hydroxyethyl)-aminobenzol, 1-Amino-2-(2'-hydroxyethyl)-amino-5-nitrobenzol (HC Yellow Nr. 5), 1-Amino-2-nitro-4-(2'-hydroxyethyl)-aminobenzol (HC Red Nr. 7), 2-Chloro-5-nitro-N-hydroxyethyl-1,4-phenylendiamin, 1-(2'-Hydroxyethyl)-amino-2-nitro-4-aminobenzol (HC Red Nr. 3), 4-Amino-3-nitrophenol, 4-Amino-2-nitrophenol, 6-Nitro-o-toluidin, 1-Amino-3-methyl-4-(2'-hydroxyethyl)-amino-6-nitrobenzol (HC Violet Nr. 1), 1-Amino-2-nitro-4-(2',3'-dihydroxypropyl)-amino-5-chlorobenzol an (HC Red Nr. 10), 2-(4'-Amino-2'-nitroanilino)-benzoesäure, 6-Nitro-2,5-diaminopyridin, 2-Amino-6-chloro-4-nitrophenol, 1-Amino-2-(3'-nitrophenylazo)-7-phenylazo-8-naphthol-3,6-disulfonsäure Dinatriumsalz (Acid blue Nr. 29), 1-Amino-2-(2'-hydroxy-4'-nitrophenylazo)-8-naphthol-3,6-disulfonsäure Dinatriumsalz (Palatinchrome green), 1-Amino-2-(3'-chlor-2'-hydroxy-5'-nitrophenylazo)-8-naphthol-3,6-disulfonsäure Dinatriumsalz (Gallion), 4-Amino-4'-nitrostilben-2,2'-disulfonsäure, Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2'-nitrobenzylidenamino)-benzol, 2-[2'-(Diethylamino)-ethylamino]-5-nitroanilin, 3-Amino-4-hydroxy-5-nitrobenzolsulfonsäure (Hydrat), 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2'-hydroxyethylamino)-benzol, 2-Nitro-1-amino-4-[bis-(2'-hydroxyethyl)-amino]-benzol, 4-Amino-2-nitrodiphenylamin-2'-carbonsäure, 1-Amino-5-chlor-4-(2'-hydroxyethylamino)-2-nitrobenzol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor -4-(2'-hydroyethylamino)-2-nitrobenzol, aromatische Aniline bzw. Phenole mit einem weiteren aromatischen Rest wie 4,4'-Diaminostilben-dihydrochlorid, 4,4'-Diaminostilben-2,2'-disulfonsäure Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraaminobenzophenon, 1,3-Bis-(2',4'-diaminophenoxy)-propan-tetrahydrochlorid, 1,8-Bis-(2',5'-diaminophenoxy)-3,6-dioxaoctan-tetrahydrochlorid, 1,3-Bis-(4'-aminophenylamino)-propan, -2-propanol, 1,3-Bis-[N-(4'-aminophenyl)-2-hydroxyethylamino]-2-propanol, N,N-Bis-[2-(4'-aminophenoxy)-ethyl]methylamin-trihydrochlorid,
stickstoffhaltigen heterocyclischen Verbindungen ausgewählt aus der Gruppe, bestehend aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethylpyrrol, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indol- und Indolinderivaten, wie 4-, 5-, 6-, 7-Aminoindol, 4-, 5-, 6-, 7-Hydroxyindol, 5,6-Dihydroxyindol, 5,6-Dihydroxyindolin, 4-Hydroxyindolin und Hydroxypyrimidinderivate, sowie jeweils aus den mit vorzugsweise anorganischen Säuren gebildeten physiologisch verträglichen Salzen dieser Verbindungen,
aromatischen Hydroxyverbindungen ausgewählt aus der Gruppe, bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2'-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, - acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure,
Aminosäuren ausgewählt aus der Gruppe β-Alanin, Arginin, Histidin, Tyrosin, Phenylalanin, DOPA (Dihydroxyphenylalanin), Omithin, Lysin, Prolin und Tryptophan,
Oligopeptid ausgewählt aus Glutathion oder die in den Hydrolysaten von Kollagen, Keratin, Casein, Elastin, Sojaprotein, Weizengluten oder Mandelprotein enthaltene Oligopeptide,
CH-aciden Verbindungen ausgewählt aus der Gruppe, bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Ethyl-2-chinaldinium-iodid, 1,4-Dimethylchinolinium-iodid, 1-Methyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon, 1-Methyl-3-phenyl-2-pyrazolinon, Indan-1,3-dion, Indan-1-on, 1-Dicyanmethylenindan
quartären Ammoniumsalzen, wie Tetramethyl-, Tetraethyl-, Tetrapropyl-, Tetrabutyl-, Benzyltrimethylammonium-chlorid, -bromid, -iodid, -hydrogensulfat, -(halb)sulfat sowie Polyquaternium 10.

7. Mittel nach Anspruch 6, **dadurch gekennzeichnet, dass** die weitere Verbindung ausgewählt ist aus der Gruppe bestehend aus N-(2'-Hydroxyethyl)-N-ethyl-, 2-Chlor p-phenylendiamin, N,N-Bis-(2'-hydroxyethyl)-p-phenylendiamin, 4-Aminophenol, 2-Amino-6-chloro-4-nitrophenol, p-Phenylendiamin, 2-(2',5'-Diaminophenyl)-ethanol, 2,5-Diaminotoluol, 3,4-Methylendioxyanilin, 2-Amino-4-(2'-hydroxyethylamino)-anisol, 2-(2',4'-Diaminophenoxy)-ethanol, 3-Amino-2,4-dichlor-, 2-Methyl-5-amino-, 3-Methyl-4-amino-, 2-Methyl-5-(2'-hydroxyethylamino)-, 2-Methyl-5-amino-4-chlor-, 6-Methyl-3-amino-2-chlor-, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triami-no-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 4,5-Diamino-1-(2-hydroxyethyl)-pyrazol, 5,6-Dihydroxyindol und 5,6-Dihydroxyindolin, Tetrabutylammoniumbromid, β-Alanin, L-Prolin, L-Lysin, DL-Tyrosin sowie jeweils aus den vorzugsweise mit anorganischen Säuren gebildeten physiologisch verträglichen Salzen dieser Verbindungen.

8. Mittel nach einem der Ansprüche 1 bis 7, **dadurch gekennzeichnet, dass** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

9. Mittel nach einem der Ansprüche 1 bis 8, **dadurch gekennzeichnet, dass** es direkt ziehende Farbstoffe aus der Gruppe der Nitrophenylendiamine, Nitroaminophenole, Anthrachinone oder Indophenole vorzugsweise in einer Menge von 0,01 bis 20 Gew.%, bezogen auf das gesamte Färbemittel, enthält.

10. Mittel nach einem der Ansprüche 1 bis 9, **dadurch gekennzeichnet, dass** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

11. Mittel nach einem der Ansprüche 1 bis 10, **dadurch gekennzeichnet, dass** es Oxidationsmittel, insbesondere H₂O₂, in einer Menge von 0,01 bis 6 Gew.%, bezogen auf die Anwendungslösung, enthält.

12. Mittel nach einem der Ansprüche 1 bis 11, **dadurch gekennzeichnet, dass** es anionische, zwitterionische oder nichtionische Tenside enthält.

13. Verwendung einer Kombination aus mindestens ein aromatisches oder heteroaromatisches Aldehyd und/oder Keton mit der Formel I oder physiologisch verträgliche Salze davon, 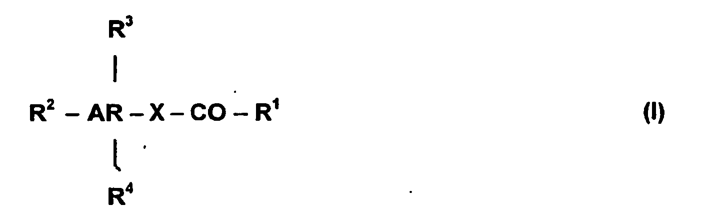 wobei
• AR steht für Benzol, Naphthalin, Pyridin, Pyrimidin, Pyrazin, Pyrazidin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
• R¹ steht für ein Wasserstoffatom, eine C₁-C₄-Alkyl-, C₁-C₅-Acyl-, C₂-C₄-Alkenyl-, C₁-C₄-Perfluoralkyl-, eine ggf. substituierte Aryl- oder Heteroarylgruppe,
• R², R³ und R⁴ stehen unabhängig voneinander für ein Wasserstoffatom, ein Halogenatom, eine C₁-C₄-Alkyl-, C₁₋₄-Alkoxy-, C₁-C₄-Aminoalkyl-, C₁-C₄-Hydroxyalkylgruppe, eine C₁-C₄-Acylgruppe, eine Carboxy-, Carboxylato-, Sulfo-, Sulfato-, C₂-C₆-Alkenyl-, Aryl-, eine Aryl-C₁-C₄-alkylgruppe, eine Hydroxy-, Nitro-, Pyrrolidino-, Morpholino-, Piperidino-, Amino- bzw. Ammonio- oder 1-Imidazol(in)iogruppe, wobei die letzten drei Gruppen mit C₁₋₄-Alkyl-, C₁₋₄-Carboxyalkyl-, C₁₋₄-Hydroxyalkyl-, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können, durch C₁-C₄-Alkyl-, C₂-C₆-Alkenyl- oder C₁-C₄-Hydroxyalkoxygruppen substituiert sein können, wobei auch zwei der Reste aus R², R³, R⁴ und -X-CO-R¹ einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R², R³ und R⁴,
• X steht für eine direkte Bindung, eine Carbonyl-, eine Carboxy-(C₁-C₄)-alkylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei X zusammen mit der CO-R¹-Gruppe auch einen gegebenenfalls substituierten 5-, 6- oder 7-Ring bilden kann,
sowie mindestens ein 4-Aminopyrazolin-5-on mit der Formel (IIa) oder (IIb) oder deren physiologisch verträgliche Salze, 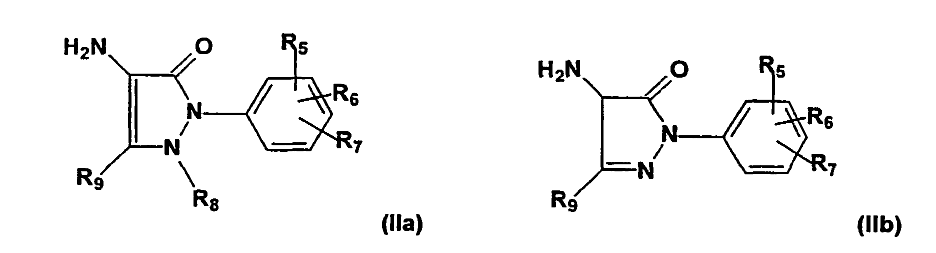 in denen
• R⁵, R⁶ und R⁷ stehen unabhängig voneinander für ein Wasserstoffatom, ein Halogenatom, eine C₁₋₄-Alkyl-, C₁₋₄-Alkoxy- oder C₁₋₄-Hydroxyalkoxygruppe, eine Hydroxy-, Carboxy-, Carboxylato-, C₁₋₄-Alkoxycarbonyl-, gegebenenfalls substituierte Carbamoyl-, Sulfo-, Sulfato-, Sulfamoylgruppe oder Aminogruppe, die durch C₁₋₄-Alkyl- oder Hydroxyalkylgruppen substituiert sein kann,
• R⁸ und R⁹ stehen unabhängig voneinander für ein Wasserstoffatom, eine Hydroxy-, Amino-, C₁₋₄-Alkyl-, C₁₋₄-Alkoxy-, Carbamoyl-, Carboxyl-, C₁₋₄-Alkoxycarbonyl-, Aryl-, Aryl-C₁₋₄-alkyl- oder Heteroarylgruppe,
als eine färbende Komponente in Oxidationshaarfärbemitteln.

14. Verfahren zum Färben von keratinhaltigen Fasern, insbesondere menschlichen Haaren, worin ein Färbemittel, enthaltend
A1) mindestens ein aromatisches oder heteroaromatisches Aldehyd und/oder Keton mit der Formel I oder physiologisch verträgliche Salze davon, 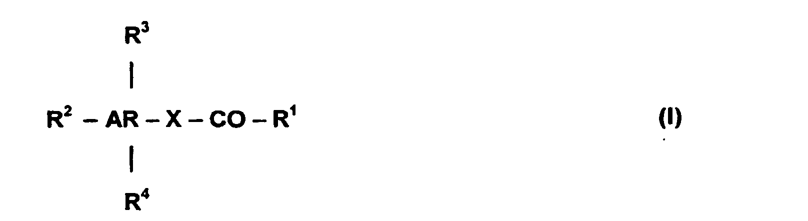 wobei
• AR steht für Benzol, Naphthalin, Pyridin, Pyrimidin, Pyrazin, Pyrazidin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
• R¹ steht für ein Wasserstoffatom, eine C₁-C₄-Alkyl-, C₁-C₅-Acyl-, C₂-C₄-Alkenyl-, C₁-C₄-Perfluoralkyl-, eine ggf. substituierte Aryl- oder Heteroarylgruppe,
• R², R³ und R⁴ stehen unabhängig voneinander für ein Wasserstoffatom, ein Halogenatom, eine C₁-C₄-Alkyl-, C₁₋₄-Alkoxy-, C₁-C₄-Aminoalkyl-, C₁-C₄-Hydroxyalkylgruppe, eine C₁-C₄-Acylgruppe, eine Carboxy-, Carboxylato-, Sulfo-, Sulfato-, C₂-C₆-Alkenyl-, Aryl-, eine Aryl-C₁-C₄-alkylgruppe, eine Hydroxy-, Nitro-, Pyrrolidino-, Morpholino-, Piperidino-, Amino- bzw. Ammonio- oder 1-Imidazol(in)iogruppe, wobei die letzten drei Gruppen mit C₁₋₄-Alkyl-, C₁₋₄-Carboxyalkyl-, C₁₋₄-Hydroxyalkyl-, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können, durch C₁-C₄-Alkyl-, C₂-C₆-Alkenyl- oder C₁-C₄-Hydroxyalkoxygruppen substituiert sein können, wobei auch zwei der Reste aus R², R³, R⁴ und X-CO-R¹ einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R², R³ und R⁴,
• X steht für eine direkte Bindung, eine Carbonyl-, eine Carboxy-(C₁-C₄)-alkylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei X zusammen mit der CO-R¹-Gruppe auch einen gegebenenfalls substituierten 5-, 6- oder 7-Ring bilden kann,
A2) sowie mindestens ein 4-Aminopyrazolin-5-on mit der Formel (IIa) oder (IIb) oder deren physiologisch verträgliche Salze, 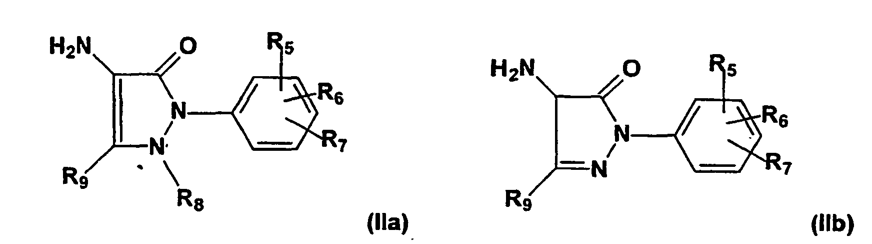 in denen
• R⁵, R⁶ und R⁷ stehen unabhängig voneinander für ein Wasserstoffatom, ein Halogenatom, eine C₁₋₄-Alkyl-, C₁₋₄-Alkoxy- oder C₁₋₄-Hydroxyalkoxygruppe, eine Hydroxy-, Carboxy-, Carboxylato-, C₁₋₄-Alkoxycarbonyl-, gegebenenfalls substituierte Carbamoyl-, Sulfo-, Sulfato-, Sulfamoylgruppe oder Aminogruppe, die durch C₁₋₄-Alkyl- oder Hydroxyalkylgruppen substituiert sein kann,
• R³ und R⁹ stehen unabhängig voneinander für ein Wasserstoffatom, eine Hydroxy-, Amino-, C₁₋₄-Alkyl-, C₁₋₄-Alkoxy-, Carbamoyl-, Carboxyl-, C₁₋₄-Alkoxycarbonyl-, Aryl-, Aryl-C₁₋₄-alkyl- oder Heteroarylgruppe und
B) und gegebenenfalls mindestens eine Verbindung mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren, aus 2 bis 9 Aminosäuren aufgebauten Oligopeptiden und/oder mindestens eine CH-acide Verbindung oder quartäre Ammoniumverbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Agent for the coloration of keratin-containing fibres, in particular human hair, comprising at least one aromatic or heteroaromatic aldehyde and/or ketone with the formula I or physiologically compatible salts thereof, 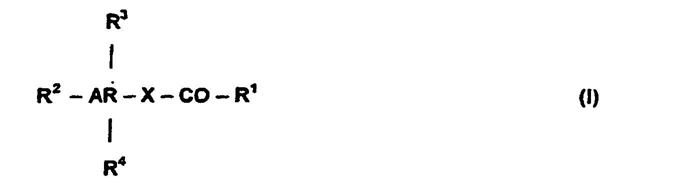 where
• AR is benzene, naphthalene, pyridine, pyrimidine, pyrazine, pyrazidine, carbazole, pyrrole, pyrazole, furan, thiophene, 1,2,3-triazine, 1,3,5-triazine, quinoline, isoquinoline, indole, indoline, indolizine, indane, imidazole, 1,2,4-triazole, 1,2,3-triazole, tetrazole, benzimidazole, 1,3-thiazole, benzothiazole, indazole, benzoxazole, quinoxaline, quinazoline, quinolizine, cinnoline, acridine, julolidine, acenaphthene, fluorene, biphenyl, diphenylmethane, benzophenone, diphenyl ether, azobenzene, chromone, coumarin, diphenylamine, stilbene, where the N-heteroaromatics may also be quaternized,
• R¹ is a hydrogen atom, a C₁-C₄-alkyl group, C₁-C₅-acyl group, C₂-C₄-alkenyl group, C₁-C₄-perfluoroalkyl group, an optionally substituted aryl group or heteroaryl group,
• R², R³ and R⁴, independently of one another, are a hydrogen atom, a halogen atom, a C₁-C₄-alkyl group, C₁₋₄-alkoxy group, C₁-C₄-aminoalkyl group, C₁-C₄-hydroxyalkyl group, a C₁-C₄-acyl group, a carboxy group, carboxylato group, sulpho group, sulphato group, C₂-C₆-alkenyl group, aryl group, an aryl-C₁-C₄-alkyl group, a hydroxy group, nitro group, pyrrolidino group, morpholino group, piperidino group, amino group or ammonio group or 1-imidazol(in)io group, where the last three groups may be substituted by C₁₋₄-alkyl groups, C₁₋₄-carboxyalkyl groups, C₁₋₄-hydroxyalkyl groups, C₂₋₄-alkenyl groups, by optionally substituted benzyl groups, by sulpho(C₁₋₄)alkyl groups or heterocycle-(C₁₋₄)-alkyl groups, may be substituted by C₁-C₄-alkyl groups, C₂-C₆-alkenyl groups or C₁-C₄-hydroxyalkoxy groups, where also two of the radicals from R², R³, R⁴ and -X-CO-R¹ can form a fused optionally substituted 5-, 6- or 7-membered ring, which can likewise carry a fused aromatic ring, where the system AR can, depending on the size of the ring, carry further substituents which, independently of one another, can be the same groups as R², R³ and R⁴,
• X is a direct bond, a carbonyl group, a carboxy (C₁-C₄)alkylene group, an optionally substituted C₂-C₆-alkenylene group, C₄-C₆-alkadienylene group, furylene group, thienylene group, arylene group, vinylenearylene group, vinylenefurylene group, vinylenethienylene group, where X together with the CO-R¹ group can also form an optionally substituted 5-, 6- or 7-membered ring, and at least one 4-aminopyrazolin-5-one with the formula (IIa) or (IIb) or physiologically compatible salts thereof, 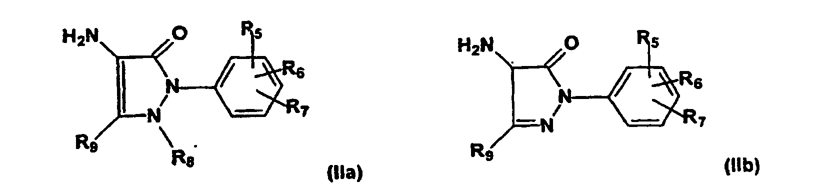
in which
• R⁵, R⁶ and R⁷, independently of one another, are a hydrogen atom, a halogen atom, a C₁₋₄-alkyl group, C₁₋₄-alkoxy group or C₁₋₄-hydroxyalkoxy group, a hydroxy group, carboxy group, carboxylato group, C₁₋₄-alkoxycarbonyl group, optionally substituted carbamoyl group, sulpho group, sulphato group, sulphamoyl group or amino group which may be substituted by C₁₋₄-alkyl or hydroxyalkyl groups,
• R⁸ and R⁹, independently of one another, are a hydrogen atom, a hydroxy group, amino group, C₁₋₄-alkyl group, C₁₋₄-alkoxy group, carbamoyl group, carboxyl group, C₁₋₄-alkoxycarbonyl group, aryl group, aryl-C₁-C₄-alkyl group or heteroaryl group.

2. Agent according to Claim 1, **characterized in that** the compounds with the formula I used are
acetophenone, propiophenone, 2-hydroxyacetophenone, 3-hydroxyacetophenone, 4-hydroxyacetophenone, 2-hydroxypropiophenone, 3-hydroxypropiophenone, 4-hydroxypropiophenone, 2-hydroxybutyrophenone, 3-hydroxybutyrophenone, 4-hydroxybutyrophenone, 2,4-dihydroxyacetophenone, 2,5-dihydroxyacetophenone, 2,6-dihydroxyacetophenone, 2,3,4-trihydroxyacetophenone, 3,4,5-trihydroxyacetophenone, 2,4,6-trihydroxyacetophenone, 2,4,6-trimethoxyacetophenone, 3,4,5-trimethoxyacetophenone, 3,4,5-trimethoxyacetophenone diethyl ketal, 4-hydroxy-3-methoxyacetophenone, 3,5-dimethoxy-4-hydroxyacetophenone, 4-aminoacetophenone, 4-dimethylaminoacetophenone, 4-morpholinoacetophenone, 4-piperidinoacetophenone, 4-imidazolinoacetophenone, 2-hydroxy-5-bromoacetophenone, 4-hydroxy-3-nitroacetophenone, acetophenone-2-carboxylic acid, acetophenone-4-carboxylic acid, benzophenone, 4-hydroxybenzophenone, 2-aminobenzophenone, 4,4'-dihydroxybenzophenone, 2,4-dihydroxybenzophenone, 2,4,4'-trihydroxybenzophenone, 2,3,4-trihydroxybenzophenone, 2-hydroxy-1-acetonaphthone, 1-hydroxy-2-acetophenone, chromone, chromone-2-carboxylic acid, flavone, 3-hydroxyflavone, 3,5,7-trihydroxyflavone, 4',5,7-trihydroxyflavone, 5,6,7-trihydroxyflavone, quercetin, 1-indanone, 9-fluorenone, 3-hydroxyfluorenone, anthrone, 1,8-dihydroxyanthrone,
salicylaldehyde, vanillin, coniferylaldehyde, 2,4-dihydroxybenzaldehyde, 4-dimethylaminobenzaldehyde, 4-diethylaminobenzaldehyde, 4-dimethylamino-2-hydroxybenzaldehyde, 4-pyrrolidinobenzaldehyde, 4-morpholinobenzaldehyde, 4-piperidinobenzaldehyde, 4-dimethylaminoacetophenone,
4-hydroxynaphthaldehyde, 4-dimethylaminonaphthaldehyde, 4-dimethylaminobenzylidene acetone, 4-dimethylaminocinnamaldehyde, 2-dimethylaminobenzaldehyde, 2-chloro-4-dimethylaminobenzaldehyde, 4-dimethylamino-2-methylbenzaldehyde, 4-diethylaminocinnamaldehyde, 4-dibutylaminobenzaldehyde, 4-diphenylaminobenzaldehyde, 2,3,6,7-tetrahydro-1H,5H-benzo[ij]quinolizine-9-carboxaldehyde, 4-dimethylamino-2-methoxybenzaldehyde, 2,3,6,7-tetrahydro-8-hydroxy-1H,5H-benzo[ij]quinolizine-9-carboxaldehyde, 4-(1-imidazolyl)benzaldehyde, 2-morpholinobenzaldehyde, indole-3-carboxaldehyde, 1-methylindole-3-carboxaldehyde, N-ethylcarbazole-3-aldehyde, 2-formylmethylene-1,3,3-trimethylindoline (Fischers aldehyde or tribase aldehyde), 2-indolealdehyde, 3-indolealdehyde, 1-methylindole-3-aldehyde, 2-methylindole-3-aldehyde, 1-acetylindole-3-aldehyde, 3-acetylindole, 1-methyl-3-acetylindole, 2-(1',3',3'-trimethyl-2'-indolinylidene)acetaldehyde, 1-methylpyrrole-2-aldehyde, 1-methyl-2-acetylpyrrole, 4-pyridinealdehyde, 2-pyridinealdehyde, 3-pyridinealdehyde, 4-acetylpyridine, 2-acetylpyridine, 3-acetylpyridine, pyridoxal, quinoline-3-aldehyde, quinoline-4-aldehyde, antipyrine-4-aldehyde, furfural, 5-nitrofurfural, 2-thenoyltrifluoroacetone, chromone-3-aldehyde, 3-(5'-nitro-2'-furyl)acrolein, 3-(2'-furyl)acrolein and imidazole-2-aldehyde, 1,3-diacetylbenzene, 1,4-diacetylbenzene, 1,3,5-triacetylbenzene, 2-benzoylacetophenone, 2-(4'-methoxybenzoyl)acetophenone, 2-(2'-furoyl)acetophenone, 2-(2'-pyridoyl)acetophenone and 2-(3'-pyridoyl)-acetophenone,
benzylidene acetone, 4-hydroxybenzylidene acetone, 2-hydroxybenzylidene acetone, 4-methoxybenzylidene acetone, 4-hydroxy-3-methoxybenzylidene acetone, 4-dimethylaminobenzylidene acetone, 3,4-methylenedioxybenzylidene acetone, 4-pyrrolidinobenzylidene acetone, 4-piperidinobenzylidene acetone, 4-morpholinobenzylidene acetone, 4-dimethylaminobenzylidene acetone, 3-benzylidene-2,4-pentane-dione, 3-(4'-hydroxybenzylidene)-2,4-pentanedione, 3-(4'-dimethylaminobenzylidene)-2,4-pentanedione, 2-benzylidenecyclohexanone, 2-(4'-hydroxybenzylidene)cyclohexanone, 2-(4'-dimethylaminobenzylidene)cyclohexanone, 3-benzylidene-1,3-cyclohexanedione, 3-(4'-hydroxybenzylidene)-1,3-cyclohexanedione, 3-(4'-dimethylaminobenzylidene)-1,3-cyclohexanedione, 3-benzylidene-5,5-dimethyl-1,3-cyclohexanedione, 3-(4'-hydroxybenzylidene)-5,5-dimethyl-1,3-cyclohexanedione, 3-(4'-hydroxy-3-methoxybenzylidene)-5,5-dimethyl-1,3-cyclohexanedione, 3-(4'-dimethylaminobenzylidene)-5,5-dimethyl-1, 3-cyclohexanedione, 2-benzylidenecyclopentanone, 2-(4'-hydroxybenzylidene)cyclopentanone, 2-(4'-dimethylaminobenzylidene)cyclopentanone,
4-dimethylaminophenylpenta-2,4-dienal, 4-diethylaminopenta-2,4-dienal, 4-methoxyphenylpenta-2,4-dienal, 3,4-dimethoxyphenylpenta-2,4-dienal, 2,4-dimethoxyphenylpenta-2,4-dienal, 4-piperidinophenylpenta-2,4-dienal, 4-morpholinophenylpenta-2,4-dienal, 4-pyrrolidinophenylpenta-2,4-dienal, 4-dimethylaminophenylhexa-2,4-dien-2-one, 4-diethylaminophenylhexa-2,4-dien-2-one, 4-methoxyphenylhexa-2,4-dien-2-one, 3,4-dimethoxyphenylhexa-2,4-dien-2-one, 2,4-dimethoxyphenylhexa-2,4-dien-2-one, 4-piperidinophenylhexa-2,4-dien-2-one, 4-morpholinophenylhexa-2,4-dien-2-one, 4-pyrrolidinophenylhexa-2,4-dien-2-one, 4-dimethylamino-1-naphthylpenta-2,4-dienal, 2-nitrobenzaldehyde, 3-nitrobenzaldehyde, 4-nitrobenzaldehyde, 4-methyl-3-nitrobenzaldehyde, 3-hydroxy-4-nitrobenzaldehyde, 4-hydroxy-3-nitrobenzaldehyde, 5-hydroxy-2-nitrobenzaldehyde, 2-hydroxy-5-nitrobenzaldehyde, 2-hydroxy-3-nitrobenzaldehyde, 2-fluoro-3-nitrobenzaldehyde, 3-methoxy-2-nitrobenzaldehyde, 4-chloro-3-nitrobenzaldehyde, 2-chloro-6-nitrobenzaldehyde, 5-chloro-2-nitrobenzaldehyde, 4-chloro-2-nitrobenzaldehyde, 2,4-benzaldehyde, 2,6-dinitrobenzaldehyde, 2-hydroxy-3-methoxy-5-nitrobenzaldehyde, 4,5-dimethoxy-2-nitrobenzaldehyde, 6-nitropiperonal, 2-nitropiperonal, 5-nitrovanillin, 2,5-dinitrosalicylaldehyde, 5-bromo-3-nitrosalicylaldehyde, 3-nitro-4-formylbenzenesulphonic acid, 4-nitro-1-naphthaldehyde, 2-nitrocinnamaldehyde, 3-nitrocinnamaldehyde, 4-nitrocinnamaldehyde, 9-methyl-3-carbazolealdehyde, 9-ethyl-3-carboxolealdehyde, 3-acetylcarbazole, 3,6-diacetyl-9-ethylcarbazole, 3-acetyl-9-methylcarbazole, 1,4-dimethyl-3-carbazolealdehyde, 1,4,9-trimethyl-3-carbazole-aldehyde,
4-trimethylammoniobenzaldehyde, 4-benzyldimethylammoniobenzaldehyde, 4-trimethylammoniocinnamaldehyde, 4-trimethylammonionaphthaldehyde, 2-methoxy-4-trimethylammoniobenzaldehyde, 4-acetylphenyltrimethylammonium, 4-(N,N-diethylmethylammonio)benzaldehyde, 4-benzoylphenyl-trimethylammonium, N,(4'-benzoylphenyl)-N,N-diethylmethylammonium, N-(4'-formylphenyl-N-methylpyrrolidinium, N-(4'-formylphenyl-N-methylpiperidinium, N-(4'-formylphenyl-N-methylmorpholinium, N-(4'-acetylphenyl-N-methylmorpholinium, N-(4'-benzoylphenyl-N-methylmorpholinium,
3-formyl-N-ethyl-N-methylcarbazolium, 1-(4'-acetylphenyl)-3-methylimidazolium, 1-(4'-acetylphenyl)-3-methyl-2-imidazolinium, 1-(4'-benzoylylphenyl)-3-methylimidazolium, 5-acetyl-1,3-diethyl-2-methylbenzimidazolium, 5-trimethylammonio-1-indanone halide, benzenesulphonate, p-toluenesulphonate, C₁-C₄-alkanesulphonate, C₁-C₄-alkyl sulphate, trifluoromethanesulphonate, perchlorate, sulphate, hydrogensulphate, tetrafluoroborate, phosphate, hexafluorophosphate or tetrachlorozincate,
4-formyl-1-methylpyridinium, 2-formyl-1-methylpyridinium, 4-formyl-1-ethylpyridinium, 2-formyl-1-ethylpyridinium, 4-formyl-1-benzylpyridinium, 2-formyl-1-benzylpyridinium, 4-formyl-1,2-dimethylpyridinium, 4-formyl-1,3-dimethylpyridinium, 4-formyl-1-methylquinolinium, 2-formyl-1-methylquinolinium, 4-acetyl-1-methylpyridinium, 2-acetyl-1-methylpyridinium, 4-acetyl-1-methylquinolinium, 5-formyl-1-methylquinolinium, 6-formyl-1-methylquinolinium, 7-formyl-1-methylquinolinium, 8-formyl-1-methylquinolinium, 5-formyl-1-ethylquinolinium, 6-formyl-1-ethylquinolinium, 7-formyl-1-ethylquinolinium, 8-formyl-1-ethylquinolinium, 5-formyl-1-benzylquinolinium, 6-formyl-1-benzylquinolinium, 7-formyl-1-benzylquinolinium, 8-formyl-1-benzylquinolinium, 5-formyl-1-allylquinolinium, 6-formyl-1-allylquinolinium, 7-formyl-1-allylquinolinium and 8-formyl-1-allylquinolinium, 5-acetyl-1-methylquinolinium, 6-acetyl-1-methylquinolinium, 7-acetyl-1-methylquinolinium, 8-acetyl-1-methylquinolinium, 5-acetyl-1-ethylquinolinium, 6-acetyl-1-ethylquinolinium, 7-acetyl-1-ethylquinolinium, 8-acetyl-1-ethylquinolinium, 5-acetyl-1-benzylquinolinium, 6-acetyl-1-benzylquinolinium, 7-acetyl-1-benzylquinolinium, 8-acetyl-1-benzylquinolinium, 5-acetyl-1-allylquinolinium, 6-acetyl-1-allylquinolinium, 7-acetyl-1-allylquinolinium and 8-acetyl-1-allylquinolinium, 9-formyl-10-methylacridinium, 4-(2'-formylvinyl)-1-methylpyridinium, 1,3-dimethyl-2-(4'-formylphenyl)benzimidazolinium, 1,3-dimethyl-2-(4'-formylphenyl)imidazolinium, 2-(4'-formylphenyl)-3-methylbenzothiazolium, 2-(4'-acetylphenyl)-3-methylbenzothiazolium,
2-(4'-formylphenyl)-3-methylbenzoxazolium, 2-(5'-formyl-2'-furyl)-3-methylbenzothiazolium, 2-(5'-formyl-2'-furyl)-3-methylbenzothiazolium, 2-(5'-formyl-2'-thienyl)-3-methylbenzothiazolium, 2-(3'-formylphenyl)-3-methylbenzothiazolium, 2-(4'-formyl-1-naphthyl)-3-methylbenzothiazolium, 5-chloro-2-(4'-formylphenyl)-3-methylbenzothiazolium, 2-(4'-formylphenyl)-3,5-dimethylbenzothiazolium, 1-methyl-2-[2-(4'-formylphenyl)-ethenyl]pyridinium, 1-methyl-4-[2-(4'-acetylphenyl)ethenyl]pyridinium, 1-benzyl-4-[2-(4'-formylphenyl)ethenyl]pyridinium, 1-methyl-4-[2-(4'-formylphenyl)ethenyl]pyridinium, 1-methyl-2-[2-(4'-formylphenyl)ethenyl]pyridinium,
1-methyl-4-[2-(4'-formylphenyl)ethenyl]-quinolinium, 1-methyl-2-[2-(4'-formylphenyl)-ethenyl]quinolinium, 1-methyl-2-[2-(5'-formyl-2'-furyl)ethenyl]quinolinium, 1-methyl-2-[2-(5'-formyl-2'-thienyl)ethenyl]quinolinium, 1-methyl-2-[2-(4'-formylphenyl)ethenyl]benzothiazolium, 1,3-dimethyl-2-[2-(4'-formylphenyl)ethenyl]benzimidazolinium, 1,3-dimethyl-2-[2-(4'-formylphenyl)-ethenyl]imidazolinium, 1-methyl-5-oxoindeno-[1,2-b]pyridinium(4-methyl-azonio-9-fluorenone), 1-ethyl-5-oxo-indeno[1,2-b]pyridinium-(4-ethyl-4-azonio-9-fluorenone), 1-benzyl-5-oxoindeno[1,2-b]-pyridinium-(4-benzyl-4-azonio-9-fluorenone),
2-methyl-5-oxo-indeno[1,2-c]pyridinium, 2-methyl-9-oxoindeno[2,1-c]pyridinium, 1-methyl-9-oxo-indeno[2,1-b]pyridinium benzenesulphonate, p-toluenesulphonate, methanesulphonate, perchlorate, sulphate, chloride, bromide, iodide, tetrachlorozincate, methyl sulphate, trifluoromethanesulphonate, tetrafluoroborate,
isatin, 1-methylisatin, 1-allylisatin, 1-hydroxymethylisatin, 5-chloroisatin, 5-methoxyisatin, 5-nitroisatin, 6-nitroisatin, 5-sulphoisatin, 5-carboxyisatin, quinisatin, 1-methylquinisatin,
acenaphthenequinone, benzil, 2,2'-furil, 2,2'-pyridil, 4,4'-pyridil, salicil, anisil, and any mixtures of the above.

3. Agent according to Claim 1 or 2, **characterized in that** the compounds with the formula IIa and IIb used are
4-amino-2,3-dimethyl-1-phenyl-3-pyrazolin-5-one (4-aminoantipyrine), 4-amino-2,3-dimethyl-1-(4'-chlorophenyl)-3-pyrazolin-5-one, 4-amino-2,3-dimethyl-1-(2'-chlorophenyl)-3-pyrazolin-5-one,
4-amino-2,3-dimethyl-1-(4'-fluorophenyl)-3-pyrazolin-5-one, 4-amino-2,3-dimethyl-1-(4'-methoxyphenyl)-3-pyrazolin-5-one, 4-amino-2,3-dimethyl-1-(p-tolyl)-3-pyrazolin-5-one, 4-amino-2-ethyl-3-methyl-1-phenyl-3-pyrazolin-5-one,
4-amino-2,3-dimethyl-1-(2',4'-dichlorophenyl)-3-pyrazolin-5-one, 4-amino-1,2-diphenyl-3-hydroxy-3-pyrazolin-5-one, 4-amino-1,2-bis(4'-chlorophenyl)-3-hydroxy-3-pyrazolin-5-one, 4-amino-3-methyl-1-phenyl-2-pyrazolin-5-one, 4-amino-3-methyl-1-(4'-sulphophenyl)-2-pyrazolin-5-one,
4-amino-3-methyl-1-(4'-methoxyphenyl)-2-pyrazolin-5-one, 4-amino-3-methyl-1-(4'-chlorophenyl)-2-pyrazolin-5-one, 4-amino-3-methyl-1-(3'-chlorophenyl)-2-pyrazolin-5-one, 4-amino-3-carbamoyl-1-phenyl-2-pyrazolin-5-one, 4-amino-3-carboxy-1-phenyl-2-pyrazolin-5-one, 4-amino-3-ethoxycarboyl-1-phenyl-2-pyrazolin-5-one, 4-amino-1-phenyl-2-pyrazolin-5-one, 4-amino-1-(4'-chlorophenyl)-2-pyrazolin-5-one, 4-amino-1-(4'-fluorophenyl)-2-pyrazolin-5-one, 4-amino-1-(4'-chlorophenyl)-2-pyrazolin-5-one, 4-amino-1-(4'-methoxyphenyl)-2-pyrazolin-5-one, 4-amino-1-(4'-carboxyphenyl)-2-pyrazolin-5-one, and their skin-compatible acid addition products.

4. Agent according to one of Claims 1 to 3, **characterized in that** the aromatic or heteroaromatic aldehydes and/or ketones of the formula I and the 4-aminopyrazolin-5-ones of the formula IIa and IIb are in each case present in an amount of from 0.03 to 65 mmol, in particular from 1 to 40 mmol, based on 100 g of the total colorant.

5. Agent according to one of Claims 1 to 4, **characterized in that** it additionally comprises at least one compound with a primary or secondary amino group or hydroxy group, chosen from primary or secondary aromatic amines, nitrogen-containing heterocyclic compounds and aromatic hydroxy compounds, amino acids, oligopeptides constructed from 2 to 9 amino acids and/or at least one CH-acidic compound and/or quaternary ammonium compound.

6. Agent according to Claim 5, **characterized in that** the further compound is chosen from primary or secondary amines from the group consisting of N-(2'-hydroxyethyl)-N-ethyl-, N-(2'-methoxyethyl)-, 2,3-, 2,4-, 2,5-dichloro-p-phenylenediamine, 2-chloro-p-phenylenediamine, N,N-bis(2'-hydroxyethyl)-p-phenylenediamine,
2,5-dihydroxy-4-morpholinoaniline dihydrobromide, 2-, 3-, 4-aminophenol, o-, m-, p-phenylenediamine, 2,4-diaminophenoxyethanol, 2-(2',5'-diaminophenyl)ethanol, 2,5-diaminotoluene, -phenol, -phenethol, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 2-methyl-5-amino-, 3-methyl-4-amino-, 2-methyl-5-(2'-hydroxyethylamino)-, 2-methyl-5-amino-4-chloro-, 6-methyl-3-amino-2-chloro-, 5-(2'-hydroxyethylamino)-4-methoxy-2-methyl-, 4-amino-2-aminomethylphenol, 2-hydroxymethyl-4-aminophenol, 2-diethylaminomethyl-4-aminophenol, 2-dimethylaminomethyl-4-aminophenol, 2,6-dichloro-9-aminophenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-aminobenzenesulphonic acid, 3-amino-4-hydroxybenzenesulphonic acid, 4-amino-3-hydroxy-naphthalenesulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-, 1,2,4-triaminobenzene, 1,2,4,5-tetraaminobenzene tetrahydrochloride, 2,4,5-triaminophenol trihydrochloride, pentaaminobenzene pentahydrochloride, hexaaminobenzene hexahydrochloride, 2,4,6-triaminoresorcinol trihydrochloride, 4,5-diaminopyrocatechin sulphate, 4,6-diaminopyrogallol dihydrochloride, 3,5-diamino-4-hydroxypyrocatechin sulphate, aromatic nitriles, amino compounds containing nitro groups, such as 3-amino-6-methylamino-2-nitropyridine, picramic acid, [8-[(4'-amino-2'-nitrophenyl)azo]-7-hydroxynaphth-2-yl]-trimethylammonium chloride, [8-[(4'-amino-3'-nitrophenyl)azo]-7-hydroxynaphth-2-yl]trimethylammonium chloride (Basic Brown 17), 1-hydroxy-2-amino-4,6-dinitrobenzene, 1-amino-2-nitro-4-bis(2'-hydroxyethyl)aminobenzene, 1-amino-2-(2'-hydroxyethyl)amino-5-nitrobenzene (HC Yellow No. 5), 1-amino-2-nitro-4-(2'-hydroxyethyl)aminobenzene (HC Red No. 7), 2-chloro-5-nitro-N-hydroxyethyl-1,4-phenylenediamine, 1-(2'-hydroxyethyl)amino-2-nitro-4-aminobenzene (HC Red No. 3), 4-amino-3-nitrophenol, 4-amino-2-nitrophenol, 6-nitro-o-toluidine, 1-amino-3-methyl-4-(2'-hydroxyethyl)amino-6-nitrobenzene (HC Violet No. 1), 1-amino-2-nitro-4-(2',3'-dihydroxypropyl)-amino-5-chlorobenzene (HC Red No. 10), 2-(4'-amino-2'-nitroanilino)benzoic acid, 6-nitro-2,5-diaminopyridine, 2-amino-6-chloro-4-nitrophenol, 1-amino-2-(3'-nitrophenylazo)-7-phenylazo-8-naphthol-3,6-disulphonic acid disodium salt (Acid Blue No. 29), 1-amino-2-(2'-hydroxy-4'-nitrophenylazo)-8-naphthol-3,6-disulphonic acid disodium salt (Palatine chrome green), 1-amino-2-(3'-chloro-2'-hydroxy-5'-nitrophenylazo)-8-naphthol-3,6-disulphonic acid disodium salt (Gallion), 4-amino-4'-nitrostilbene-2,2'-disulphonic acid, disodium salt, 2,4-diamino-3',5'-dinitro-2'-hydroxy-5-methylazobenzene (Mordant Brown 4), 4'-amino-4-nitrodiphenylamine-2-sulphonic acid, 4'-amino-3'-nitrobenzophenone-2-carboxylic acid, 1-amino-4-nitro-2-(2'-nitrobenzylideneamino)benzene, 2-[2'-(diethylamino)ethylamino]-5-nitroaniline, 3-amino-4-hydroxy-5-nitrobenzenesulphonic acid (hydrate), 3-amino-3'-nitrobiphenyl, 3-amino-4-nitroacenaphthene, 2-amino-1-nitronaphthalene, 5-amino-6-nitrobenzo-1,3-dioxol, anilines, in particular anilines containing nitro groups, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2'-hydroxyethylamino)benzene, 2-nitro-1-amino-4-[bis(2'-hydroxyethyl)amino]benzene, 4-amino-2-nitrodiphenylamine-2'-carboxylic acid, 1-amino-5-chloro-4-(2'-hydroxyethylamino)-2-nitrobenzene,
anilines, in particular anilines containing nitro groups, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2-hydroxyethylamino)benzene, 2-nitro-1-amino-4-[bis-(2-hydroxyethyl)amino]benzene, 4-amino-2-nitrodiphenylamine-2'-carboxylic acid, 1-amino-5-chloro-4-(2'-hydroxyethylamino)-2-nitrobenzene,
aromatic anilines and phenols with a further aromatic radical, such as 4,4'-diaminostilbene dihydrochloride, 4,4'-diaminostilbene-2,2-disulphonic acid Na salt, 4,4'-diaminodiphenylmethane, sulphide, sulphoxide, amine, 4,4'-diaminodiphenylamine-2-sulphonic acid, 4,4'-diaminobenzophenone, -diphenyl ether, 3,3',4,4'-tetraaminodiphenyl tetrahydrochloride, 3,3',4,4'-tetraaminobenzophenone, 1,3-bis(2',4'-diaminophenoxy)propane tetrahydrochloride, 1,8-bis(2',5'-diaminophenoxy)-3,6-dioxaoctane tetrahydrochloride, 1,3-bis(4'-aminophenylamino)propane, -2-propanol, 1,3-bis([N-(4'-aminophenyl)-2-hydroxyethylamino]-2-propanol, N,N-bis[2-(4'-aminophenoxy)ethyl]methylamine trihydrochloride,
heterocyclic compounds containing nitrogen chosen from the group consisting of 2-, 3-, 4-amino-, 2-amino-3-hydroxy-, 2,6-diamino-, 2,5-diamino-, 2,3-diamino-, 2-dimethylamino-5-amino-, 3-amino-2-methylamino-6-methoxy-, 2,3-diamino-6-methoxy-, 3,5-diamino-2,6-dimethoxy-, 2,4,5-triamino-, 2,6-dihydroxy-3,4-dimethylpyridine,
4,5,6-triamino-, 2-hydroxy-4,5,6-triamino-, 4-hydroxy-2,5,6-triamino-, 2,4,5,6-tetraamino-, 2-methylamino-4,5,6-triamino-, 2,4-, 4,5-diamino-, 2-amino-4-methoxy-6-methylpyrimidine, 2,3,4-trimethylpyrrole, 2,4-dimethyl-3-ethylpyrrole, 3,5-diaminopyrazole, -1,2,4-triazole, 3-amino-, 3-amino-5-hydroxypyrazole, 2-, 3-, 8-aminoquinoline, 4-aminoquinaldine, 2-, 6-aminonicotinic acid, 5-aminoisoquinoline, 5-, 6-aminoindazole, 5-, 7-aminobenzimidazole, -benzothiazole, 2,5-dihydroxy-4-morpholinoaniline, and indole and indoline derivatives, such as 4-, 5-, 6-, 7-aminoindole, 4-, 5-, 6-, 7-hydroxyindole, 5,6-dihydroxyindole, 5,6-dihydroxyindoline, 4-hydroxyindoline and hydroxypyrimidine derivatives, and in each case physiologically compatible salts of these compounds formed with preferably inorganic acids,
aromatic hydroxy compounds chosen from the group consisting of 2-, 4-, 5-methylresorcinol, 2,5-dimethylresorcinol, resorcinol, 3-methoxyphenol, pyrocatechin, hydroquinone, pyrogallol, phloroglucine, hydroxyhydroquinone, 2-, 3-, 4-methoxy, 3-dimethylamino-, 2-(2'-hydroxyethyl)-, 3,4-methylenedioxyphenol, 2,4-, 3,4-dihydroxybenzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalenesulphonic acid, 3,6-dihydroxy-2,7-naphthalenesulphonic acid,
amino acids chosen from the group β-alanine, arginine, histidine, tyrosine, phenylalanine, DOPA (dihydroxyphenylalanine), ornithine, lysine, proline and tryptophan,
oligopeptide chosen from glutathione or the oligopeptides present in the hydrolysates of collagen, keratin, casein, elastin, soja protein, wheat gluten or almond protein,
CH-acidic compounds chosen from the group consisting of 1,2,3,3-tetramethyl-3H-indolium iodide, 1,2,3,3-tetramethyl-3H-indolium p-toluenesulphonate, 1,2,3,3-tetramethyl-3H-indolium methanesulphonate, 1,3,3-trimethyl-2-methyleneindoline (Fischers base), 2,3-dimethylbenzothiazolium iodide, 2,3-dimethylbenzothiazolium p-toluenesulphonate, 1,2-dimethylnaphtho[1,2-d]-thiazolium p-toluenesulphonate, 1-ethyl-2-methylnaphtho[1,2-d]thiazolium p-toluenesulphonate, rhodanine, rhodanine-3-acetic acid, 1-ethyl-2-quinaldinium iodide, 1,4-dimethylquinolinium iodide, 1-methyl-2-quinaldinium iodide, 1,4-dimethylquinolinium iodide, barbituric acid, thiobarbituric acid, 1,3-dimethylthiobarbituric acid, 1,3-diethylthiobarbituric acid, diethylthiobarbituric acid, oxindole, 3-indoxyl acetate, coumaranone, 1-methyl-3-phenyl-2-pyrazolinone, indane-1,3-dione, indan-1-one, 1-dicyanomethylene-indane
quaternary ammonium salts, such as tetramethyl-, tetraethyl-, tetrapropyl-, tetrabutyl-, benzyltrimethylammonium chloride, bromide, iodide, hydrogensulphate, (half-)sulphate
and polyquaternium 10.

7. Agent according to Claim 6, **characterized in that** the further compound is chosen from the group consisting of N-(2'-hydroxyethyl)-N-ethyl-, 2-chloro-p-phenylenediamine, N,N-bis(2'-hydroxyethyl)-p-phenylenediamine, 4-aminophenol, 2-amino-6-chloro-4-nitrophenol, p-phenylenediamine, 2-(2',5'-diaminophenyl)ethanol, 2,5-diaminotoluene, 3,4-methylenedioxyaniline, 2-amino-4-(2'-hydroxyethylamino)anisole, 2-(2',4'-diaminophenoxy)ethanol, 3-amino-2,4-dichloro-, 2-methyl-5-amino-, 3-methyl-4-amino-, 2-methyl-5-(2'-hydroxyethylamino)-, 2-methyl-5-amino-4-chloro-, 6-methyl-3-amino-2-chloro-, 2-aminomethyl-4-aminophenol, 2-diethylaminomethyl-4-aminophenol, 2-dimethylaminomethyl-4-aminophenol, 2,6-dichloro-4-aminophenol, 2-hydroxymethyl-4-aminophenol, 3,4-methylenedioxyphenol, 3,4-diaminobenzoic acid, 2,5-diamino-, 2-dimethylamino-5-amino-, 3-amino-2-methylamino-6-methoxy-, 2,3-diamino-6-methoxy-, 3,5-diamino-2,6-dimethoxy-, 2,6-dihydroxy-3,4-dimethylpyridine, 2-hydroxy-4,5,6-triamino-, 4-hydroxy-2,5,6-triamino-, 2,4,5,6-tetraamino-, 2-methylamino-4,5,6-triaminopyrimidine,
3,5-diaminopyrazole, 3-amino-5-hydroxypyrazole, 4,5-diamino-1-(2-hydroxyethyl)pyrazole, 5,6-dihydroxyindole and 5,6-dihydroxyindoline, tetrabutylammonium bromide, β-alanine, L-proline, L-lysine, DL-tyrosine and in each case from the physiologically compatible salts of these compounds formed preferably with inorganic acids.

8. Agent according to one of Claims 1 to 7, **characterized in that** it comprises colour enhancers chosen from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazidine or any mixtures thereof.

9. Agent according to one of Claims 1 to 8, **characterized in that** it comprises direct dyes from the group of nitrophenylenediamines, nitroaminophenols, anthraquinones or indophenols preferably in an amount of from 0.01 to 20% by weight, based on the total colorant.

10. Agent according to one of Claims 1 to 9, **characterized in that** ammonium or metal salts chosen from the group of formates, carbonates, halides, sulphates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc, are added.

11. Agent according to one of Claims 1 to 10, **characterized in that** it comprises oxidizing agents, in particular H₂O₂, in an amount of from 0.01 to 6% by weight, based on the application solution.

12. Agent according to one of Claims 1 to 11, **characterized in that** it comprises anionic, zwitterionic or nonionic surfactants.

13. Use of a combination of at least one aromatic or heteroaromatic aldehyde and/or ketone with the formula I or physiologically compatible salts thereof, 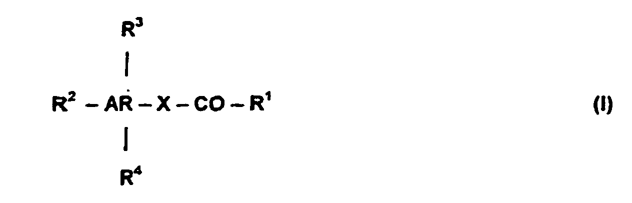 where
• AR is benzene, naphthalene, pyridine, pyrimidine, pyrazine, pyrazidine, carbazole, pyrrole, pyrazole, furan, thiophene, 1,2,3-triazine, 1,3,5-triazine, quinoline, isoquinoline, indole, indoline, indolizine, indane, imidazole, 1,2,4-triazole, 1,2,3-triazole, tetrazole, benzimidazole, 1,3-thiazole, benzothiazole, indazole, benzoxazole, quinoxaline, quinazoline, quinolizine, cinnoline, acridine, julolidine, acenaphthene, fluorene, biphenyl, diphenylmethane, benzophenone, diphenyl ether, azobenzene, chromone, coumarin, diphenylamine, stilbene, where the N-heteroaromatics may also be quaternized,
• R¹ is a hydrogen atom, a C₁-C₄-alkyl group, C₁-C₅-acyl group, C₂-C₄-alkenyl group, C₁-C₄-perfluoroalkyl group, an optionally substituted aryl group or heteroaryl group,
• R², R³ and R⁴, independently of one another, are a hydrogen atom, a halogen atom, a C₁-C₄-alkyl group, C₁₋₄-alkoxy group, C₁-C₄-aminoalkyl group, C₁-C₄-hydroxyalkyl group, a C₁-C₄-acyl group, a carboxy group, carboxylato group, sulpho group, sulphato group, C₂-C₆-alkenyl group, aryl group, an aryl-C₁-C₄-alkyl group, a hydroxy group, nitro group, pyrrolidino group, morpholino group, piperidino group, amino group or ammonio group or 1-imidazol(in)io group, where the last three groups may be substituted by C₁₋₄-alkyl groups, C₁₋₄-carboxyalkyl groups, C₁₋₄-hydroxyalkyl groups, C₂₋₄-alkenyl groups, by optionally substituted benzyl groups, by sulpho(C₁₋₄)alkyl groups or heterocycle-(C₁₋₄)-alkyl groups, may be substituted by C₁-C₄-alkyl groups, C₂-C₆-alkenyl groups or C₁-C₄-hydroxyalkoxy groups, where also two of the radicals from R², R³, R⁴ and -X-CO-R¹ can form a fused optionally substituted 5-, 6- or 7-membered ring, which can likewise carry a fused aromatic ring, where the system AR can, depending on the size of the ring, carry further substituents which, independently of one another, can be the same groups as R², R³ and R⁴,
• X is a direct bond, a carbonyl group, a carboxy(C₁-C₄)alkylene group, an optionally substituted C₂-C₆-alkenylene group, C₄-C₆-alkadienylene group, furylene group, thienylene group, arylene group, vinylenearylene group, vinylenefurylene group, vinylenethienylene group, where X together with the CO-R¹ group can also form an optionally substituted 5-, 6- or 7-membered ring,
and at least one 4-aminopyrazolin-5-one with the formula (IIa) or (IIb) or physiologically compatible salts thereof, 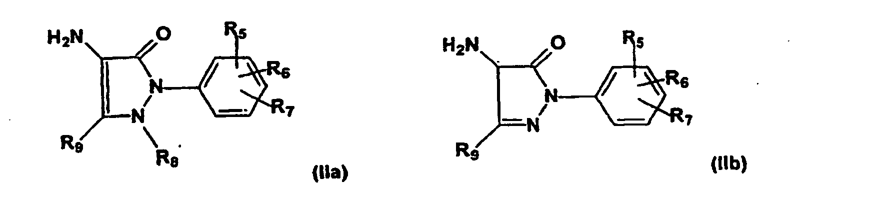 in which
• R⁵, R⁶ and R⁷, independently of one another, are a hydrogen atom, a halogen atom, a C₁₋₄-alkyl group, C₁₋₄-alkoxy group or C₁₋₄-hydroxyalkoxy group, a hydroxy group, carboxy group, carboxylato group, C₁₋₄-alkoxycarbonyl group, optionally substituted carbamoyl group, sulpho group, sulphato group, sulphamoyl group or amino group which may be substituted by C₁₋₄-alkyl or hydroxyalkyl groups,
• R⁸ and R⁹, independently of one another, are a hydrogen atom, a hydroxy group, amino group, C₁₋₄-alkyl group, C₁₋₄-alkoxy group, carbamoyl group, carboxyl group, C₁₋₄-alkoxycarbonyl group, aryl group, aryl-C₁-C₄-alkyl group or heteroaryl group, as a colouring component in oxidation hair colorants.

14. Method for the coloration of keratin-containing fibres, in particular human hair, in which a colorant comprising
A1) at least one aromatic or heteroaromatic aldehyde and/or ketone with the formula I or physiologically compatible salts thereof, 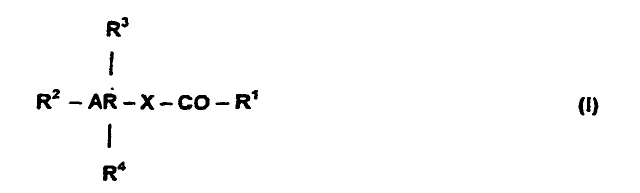 where
• AR is benzene, naphthalene, pyridine, pyrimidine, pyrazine, pyrazidine, carbazole, pyrrole, pyrazole, furan, thiophene, 1,2,3-triazine, 1,3,5-triazine, quinoline, isoquinoline, indole, indoline, indolizine, indane, imidazole, 1,2,4-triazole, 1,2,3-triazole, tetrazole, benzimidazole, 1,3-thiazole, benzothiazole, indazole, benzoxazole, quinoxaline, quinazoline, quinolizine, cinnoline, acridine, julolidine, acenaphthene, fluorene, biphenyl, diphenylmethane, benzophenone, diphenyl ether, azobenzene, chromone, coumarin, diphenylamine, stilbene, where the N-heteroaromatics may also be quaternized,
• R¹ is a hydrogen atom, a C₁-C₄-alkyl group, C₁-C₅-acyl group, C₂-C₄-alkenyl group, C₁-C₄-perfluoroalkyl group, an optionally substituted aryl group or heteroaryl group,
• R², R³ and R⁴, independently of one another, are a hydrogen atom, a halogen atom, a C₁-C₄-alkyl group, C₁₋₄-alkoxy group, C₁-C₄-aminoalkyl group, C₁-C₄-hydroxyalkyl group, a C₁-C₄-acyl group, a carboxy group, carboxylato group, sulpho group, sulphato group, C₂-C₆-alkenyl group, aryl group, an aryl-C₁-C₄-alkyl group, a hydroxy group, nitro group, pyrrolidino group, morpholino group, piperidino group, amino group or ammonio group or 1-imidazol(in)io group, where the last three groups may be substituted by C₁₋₄-alkyl groups, C₁₋₄-carboxyalkyl groups, C₁₋₄-hydroxyalkyl groups, C₂₋₄-alkenyl groups, by optionally substituted benzyl groups, by sulpho(C₁₋₄)alkyl groups or heterocycle-(C₁₋₄)-alkyl groups, may be substituted by C₁-C₄-alkyl groups, C₂-C₆-alkenyl groups or C₁-C₄-hydroxyalkoxy groups, where also two of the radicals from R², R³, R⁴ and -X-CO-R¹ can form a fused optionally substituted 5-, 6- or 7-membered ring, which can likewise carry a fused aromatic ring, where the system AR can, depending on the size of the ring, carry further substituents which, independently of one another, can be the same groups as R², R³ and R⁴,
• X is a direct bond, a carbonyl group, a carboxy(C₁-C₄)alkylene group, an optionally substituted C₂-C₆-alkenylene group, C₄-C₆-alkadienylene group, furylene group, thienylene group, arylene group, vinylenearylene group, vinylenefurylene group, vinylenethienylene group, where X together with the CO-R¹ group can also form an optionally substituted 5-, 6- or 7-membered ring,
A2) and at least one 4-aminopyrazolin-5-one with the formula (IIa) or (IIb) or physiologically compatible salts thereof, 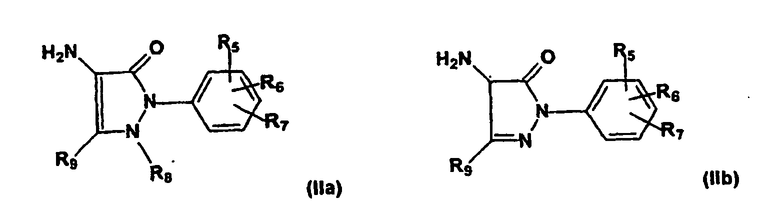 in which
• R⁵, R⁶ and R⁷, independently of one another, are a hydrogen atom, a halogen atom, a C₁₋₄-alkyl group, C₁₋₄-alkoxy group or C₁₋₄-hydroxyalkoxy group, a hydroxy group, carboxy group, carboxylato group, C₁₋₄-alkoxycarbonyl group, optionally substituted carbamoyl group, sulpho group, sulphato group, sulphamoyl group or amino group which may be substituted by C₁₋₄-alkyl or hydroxyalkyl groups,
• R⁸ and R⁹, independently of one another, are a hydrogen atom, a hydroxy group, amino group, C₁₋₄-alkyl group, C₁₋₄-alkoxy group, carbamoyl group, carboxyl group, C₁₋₄-alkoxycarbonyl group, aryl group, aryl-C₁-C₄-alkyl group or heteroaryl group,
B) and optionally at least one compound with a primary or secondary amino group or hydroxy group, chosen from primary or secondary aromatic amines, nitrogen-containing heterocyclic compounds and aromatic hydroxy compounds, amino acids, oligopeptides constructed from 2 to 9 amino acids and/or at least one CH-acidic compound or and quaternary ammonium compound,
and customary cosmetic ingredients, is applied to the keratin-containing fibres, left on the fibres for a certain amount of time, usually about 30 minutes, and then rinsed out again or washed out with a shampoo.

## Revendications

1. Agent pour la coloration de fibres kératiniques, en particulier de cheveux humains, contenant au moins un aldéhyde et/ou une cétone aromatique ou hétéroaromatique répondant à la formule I ou encore leurs sels physiologiquement acceptables 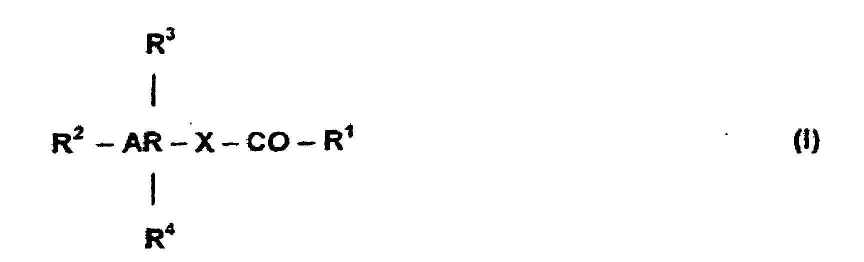 formule dans laquelle
• AR représente un groupe benzène, un groupe naphtalène, un groupe pyridine, un groupe pyrimidine, un groupe pyrazine, un groupe pyrazidine, un groupe carbazole, un groupe pyrrole, un groupe pyrazole, un groupe furanne, un groupe thiophène, un groupe 1,2,3-triazine, un groupe 1,3,5-triazine, un groupe quinoléine, un groupe isoquinoléine, un groupe indole, un groupe indoline, un groupe indolizine, un groupe indane, un groupe imidazole, un groupe 1,2,4-triazole, un groupe 1,2,3-triazole, un groupe tétrazole, un groupe benzimidazole, un groupe 1,3-thiazole, un groupe benzothiazole, un groupe indazole, un groupe benzoxazole, un groupe quinoxaline, un groupe quinazoline, un groupe quinolizine, un groupe cinnoline, un groupe acridine, un groupe julolidine, un groupe acénaphtène, un groupe fluorène, un groupe biphényle, un groupe diphénylméthane, un groupe benzophénone, un groupe diphényléther, un groupe azobenzène, un groupe chromone, un groupe coumarine, un groupe diphénylamine, un groupe stilbène, les composés hétéroaromatiques azotés pouvant également être quaternisés,
• R¹ représente un atome d'hydrogène, un groupe alkyle en C₁-C₄, un groupe acyle en C₁-C₅, un groupe alcényle en C₂-C₄, un groupe perfluoroalkyle en C₁-C₄, un groupe aryle ou hétéroaryle éventuellement substitué,
• R², R³ et R⁴ représentent, indépendamment l'un de l'autre, un atome d'hydrogène ; un atome d'halogène ; un groupe alkyle en C₁-C₄, un groupe alcoxy en C₁-C₄; un groupe aminoalkyle en C₁-C₄, un groupe hydroxyalkyle en C₁-C₄; un groupe acyle en C₁-C₄ ; un groupe carboxyle, un groupe carboxylato, un groupe sulfo, un groupe sulfato, un groupe alcényle en C₂-C₆, un groupe aryle ; un groupe arylalkyle en C₁-C₄ ; un groupe hydroxyle, un groupe nitro, un groupe pyrrolidino, un groupe morpholino, un groupe pipéridino, un groupe amino, respectivement un groupe ammonio ou un groupe 1-imidazol(in)io, les trois derniers groupes mentionnés pouvant être substitués avec un groupe alkyle en C₁-C₄, avec un groupe carboxyalkyle en C₁-C₄, avec un groupe hydroxyalkyle en C₁-C₄, avec des groupes alcényle en C₂-C₄, avec des groupes benzyle éventuellement substitués, avec des groupes sulfoalkyle en C₁-C₄ ou avec des groupes alkyle en C₁-C₄ contenant un noyau hétérocyclique, qui peuvent être substitués par un groupe alkyle en C₁-C₄, par un groupe alcényle en C₂-C₆ ou par un groupe hydroxyalcoxy en C₁-C₄, deux des radicaux choisis parmi le groupe comprenant R², R³, R⁴ et -X-CO-R¹ pouvant également former un noyau condensé à 5 membres, à 6 membres ou à 7 membres, éventuellement substitué, qui peut également porter un noyau aromatique condensé, le système AR, en fonction de la dimension du noyau pouvant porter d'autres substituants qui peuvent représenter, indépendamment les uns des autres, les mêmes substituants que ceux indiqués pour R², R³ et R⁴,
• X représente une liaison directe ; un groupe carbonyle ; un groupe carboxyalkylène en C₁-C₄ ; un groupe alcénylène en C₂-C₆, un groupe alcadiénylène en C₄-C₆, un groupe furylène, un groupe thiénylène, un groupe arylène, un groupe vinylènearylène, un groupe vinylènefurylène, un groupe vinylènethiénylène, ces groupes étant éventuellement substitués ; X peut également former, ensemble avec le groupe CO-R¹, un noyau à 5 membres, à 6 membres ou à 7 membres, éventuellement substitué,
et au moins une 4-aminopyrazolin-5-one répondant à la formule (IIa) ou à la formule (IIb) ou ses sels physiologiquement acceptables, 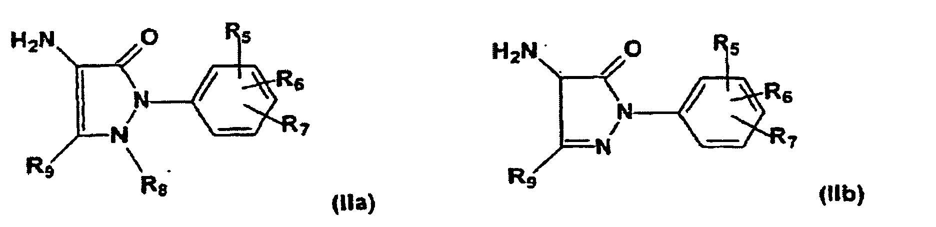 formules dans lesquelles
• R⁵, R⁶ et R⁷ représentent, indépendamment l'un de l'autre, un atome d'hydrogène, un atome d'halogène, un groupe alkyle en C₁-C₄, un groupe alcoxy en C₁-C₄ ou un groupe hydroxyalcoxy en C₁-C₄; un groupe hydroxyle, un groupe carboxyle, un groupe carboxylato, un groupe alcoxy(en C₁-C₄)carbonyle, un groupe carbamoyle éventuellement substitué, un groupe sulfo, un groupe sulfato, un groupe sulfamoyle ou un groupe amino, qui peut être substitué par des groupes alkyle ou hydroxyalkyle en C₁-C₄,
• R⁸ et R⁹ représentent, indépendamment l'un de l'autre, un atome d'hydrogène ; un groupe hydroxyle, un groupe amino, un groupe alkyle en C₁-C₄, un groupe alcoxy en C₁-C₄, un groupe carbamoyle, un groupe carboxyle, un groupe alcoxy(en C₁-C₄)-carbonyle, un groupe aryle, un groupe arylalkyle en C₁-C₄ ou un groupe hétéroaryle.

2. Agent selon la revendication 1, **caractérisé en ce qu'**on met en oeuvre, à titre de composés répondant à la formule I, l'acétophénone, la propiophénone, la 2-hydroxyacétophénone, la 3-hydroxyacétophénone, la 4-hydroxyacétophénone, la 2-hydroxypropiophénone, la 3-hydroxypropiophénone, la 4-hydroxypropiophénone, la 2-hydroxybutyrophénone, la 3-hydroxybutyrophénone, la 4-hydroxybutyrophénone, la 2,4-dihydroxyacétophénone, la 2,5-dihydroxyacétophénone, la 2,6-dihydroxyacétophénone, la 2,3,4-trihydroxyacétophénone, la 3,4,5-trihydroxyacétophénone, la 2,4,6-trihydroxyacétophénone, la 2,4,6-triméthoxyacétophénone, la 3,4,5-triméthoxyacétophénone, le diéthylacétal de 3,4,5-triméthoxyacétophénone, la 4-hydroxy-3-méthoxy-acétophénone, la 3,5-diméthoxy-4-hydroxyacétophénone, la 4-aminoacétophénone, la 4-diméthylaminoacétophénone, la 4-morpholinoacétophénone, la 4-pipéridinoacétophénone, la 4-imidazolinoacétophénone, la 2-hydroxy-5-bromoacétophénone, la 4-hydroxy-3-nitroacétophénone, l'acide acétophénone-2-carboxylique, l'acide acétophénone-4-carboxylique, la benzophénone, la 4-hydroxybenzophénone, la 2-aminobenzophénone, la 4,4'-dihydroxybenzophénone, la 2,4-dihydroxybenzophénone, la 2,4,4'-trihydroxybenzophénone, la 2,3,4-trihydroxybenzophénone, la 2-hydroxy-1-acétonaphtone, la 1-hydroxy-2-acétonaphtone, la chromone, l'acide chromone-2-carboxylique, la flavone, la 3-hydroxyflavone, la 3,5,7-trihydroxyflavone, la 4',5,7-trihydroxyflavone, la 5,6,7-trihydroxyflavone, la quercétine, la 1-indanone, la 9-fluorénone, la 3-hydroxyfluorénone, l'anthrone, la 1,8-dihydroxyanthrone,
le salicylaldéhyde, la vanilline, le coniférylaldéhyde, le 2,4-dihydroxybenzaldéhyde, le 4-diméthylaminobenzaldéhyde, le 4-diéthylaminobenzaldéhyde, le 4-diméthylamino-2-hydroxybenzaldéhyde, le 4-pyrrolidinobenzaldéhyde, le 4-morpholinobenzaldéhyde, le 4-pipéridinobenzaldéhyde, la 4-diméthylaminoacétophénone, le 4-hydroxynaphtaldéhyde, le 4-diméthylaminonaphtaldéhyde, la 4-diméthylaminobenzylidèneacétone, le 4-diméthylaminocinnamaldéhyde, le 2-diméthylaminobenzaldéhyde, le 2-chloro-4-diméthylaminobenzaldéhyde, le 4-diméthylamino-2-méthylbenzaldéhyde, le 4-diéthylaminocinnamaldéhyde, le 4-dibutylaminobenzaldéhyde, le 4-diphénylaminobenzaldéhyde, le 2,3,6,7-tétrahydro-1H,5H-benzo[ij]-quinolizine-9-carboxaldéhyde, le 4-diméthylamino-2-méthoxybenzaldéhyde, le 2,3,6,7-tétrahydro-8-hydroxy-1H,5H-benzo[ij]-quinolizine-9-carboxaldéhyde, le 4-(1-imidazolyl)benzaldéhyde, le 2-morpholinobenzaldéhyde, l'indol-3-carboxaldéhyde, le 1-méthylindol-3-carboxaldéhyde, le N-éthyl-carbazol-3-aldéhyde, la 2-formyl-méthylène-1,3,3-triméthylindoline (aldéhyde de Fischer ou aldéhyde tribasique),
le 2-indolaldéhyde, le 3-indolaldéhyde, le 1-méthylindol-3-aldéhyde, le 2-méthylindol-3-aldéhyde, le 1-acétylindol-3-aldéhyde, le 3-acétylindole, le 1-méthyl-3-acétylindole, le 2-(1',3',3'-triméthyl-2'-indolinylidène)-acétaldéhyde, le 1-méthylpyrrol-2-aldéhyde, le 1-méthyl-2-acétylpyrrole, le 4-pyridinaldéhyde, le 2-pyridinaldéhyde, le 3-pyridinaldéhyde, la 4-acétylpyridine, la 2-acétylpyridine, la 3-acétylpyridine, le pyridoxal, le quinoléine-3-aldéhyde, le quinoléine-4-aldéhyde, l'antipyrine-4-aldéhyde, le furfural, le 5-nitro-furfural, la 2-thénoyl-trifluoroacétone, le chromone-3-aldéhyde, la 3-(5'-nitro-2'-furyl)-acroléine, la 3-(2'-furyl)-acroléine et l'imidazol-2-aldéhyde,
le 1,3-diacétylbenzène, le 1,4-diacétylbenzène, le 1,3,5-triacétylbenzène, la 2-benzoylacétophénone, la 2-(4'-méthoxybenzoyl)acétophénone, la 2-(2'-furoyl)acétophénone, la 2-(2'-pyridoyl)acétophénone et la 2-(3'-pyridoyl)acétophénone,
la benzylidène-acétone, la 4-hydroxybenzylidène-acétone, la 2-hydroxybenzylidène-acétone, la 4-méthoxybenzylidène-acétone, la 4-hydroxy-3-méthoxybenzylidène-acétone, la 4-diméthylaminobenrylidène-acétone, la 3,4-méthylènedioxybenzylidène-acétone, la 4-pyrrolidinobenzylidène-acétone, la 4-pipéridinobenzylidène-acétone, la 4-morpholino-benzylidène-acétone, la 4-diéthylaminobenzylidène-acétone, la 3-benzylidène-2,4-pentanedione, la 3-(4'-hydroxybenzylidène)-2,4-pentanedione, la 3-(4'-diméthylaminobenzylidène)-2,4-pentanedione, la 2-benzylidènecyclohexanone, la 2-(4'-hydroxybenzylidène)-cyclohexanone, la 2-(4'-diméthylaminobenzylidène)-cyclohexanone, la 3-benzylidène-1,3-cyclohexanedione, la 3-(4'-hydroxybenzylidène)-1,3-cyclohexanedione, la 3-(4'-diméthylaminobenzylidène)-1,3-cyclohexane-dione, la 3-benzylidène-5,5-diméthyl-1,3-cyclohexanedione, la 3-(4'-hydroxybenzylidène)-5,5-diméthyl-1,3-cyclohexanedione, la 3-(4'-hydroxy-3-méthoxybenzylidène)-5,5-diméthyl-1,3-cyclohexanedione, la 3-(4'-diméthylaminobenzylidène)-5,5-diméthyl-1,3-cyclohexanedione, la 2-benzylidènecyclopentanone, la 2-(4'-hydroxybenzylidène)-cyclo-pentanone, la 2-(4'-diméthylaminobenzylidène)-cyclopentanone, le 4-diméthylaminophénylpenta-2,4-diénal, le 4-diéthylaminophénylpenta-2,4-diénal, le 4-méthoxyphénylpenta-2,4-diénal, le 3,4-diméthoxyphényl-penta-2,4-diénal, le 2,4-diméthoxyphénylpenta-2,4-diénal, le 4-pipéridinophénylpenta-2,4-diénal, le 4-morpholinophénylpenta-2,4-diénal, le 4-pyrrolidinophénylpenta-2,4-diénal,
la 4-diméthylaminophénylhexa-2,4-dién-2-one, la 4-diéthylaminophénylhexa-2,4-dién-2-one, la 4-méthoxyphénylhexa-2,4-dién-2-one, la 3,4-diméthoxyphénylhexa-2,4-dién-2-one, la 2,4-diméthoxyphénylhexa-2,4-dién-2-one, la 4-pipéridino-phénylhexa-2,4-dién-2-one, la 4-morpholinophénylhexa-2,4-dién-2-one, la 4-pyrrolidinophénylhexa-2,4-dién-2-one, le 4-diméthylamino-1-naphtyl-penta-2,4-diénal, le 2-nitrobenzaldéhyde, le 3-nitrobenzaldéhyde, le 4-nitrobenzaldéhyde, le 4-méthyl-3-nitrobenzaldéhyde, le 3-hydroxy-4-nitrobenzaldéhyde, le 4-hydroxy-3-nitrobenzaldéhyde, le 5-hydroxy-2-nitrobenzaldéhyde, le 2-hydroxy-5-nitrobenzaldéhyde, le 2-hydroxy-3-nitrobenzaldéhyde, le 2-fluoro-3-nitrobenzaldéhyde, le 3-méthoxy-2-nitrobenzaldéhyde, le 4-chloro-3-nitrobenzaldéhyde, le 2-chloro-6-nitrobenzaldéhyde, le 5-chloro-2-nitrobenzaldéhyde, le 4-chloro-2-nitrobenzaldéhyde, le 2,4-benzaldéhyde, le 2,6-dinitrobenzaldéhyde, le 2-hydroxy-3-méthoxy-5-nitrobenzaldéhyde, le 4,5-diméthoxy-2-nitrobenzaldéhyde, le 6-nitropipéronal, le 2-nitropipéronal, la 5-nitrovaniline, le 2,5-dinitrosalicylaldéhyde, le 5-bromo-3-nitro-salicylaldéhyde, l'acide 3-nitro-4-formylbenzènesulfonique, le 4-nitro-1-naphtatdéhyde, le 2-nitrocinnamaldéhyde, le 3-nitrocinnamaldéhyde, le 4-nitrocinnamaldéhyde, le 9-méthyl-3-carbazolaldéhyde, le 9-éthyl-3-carbazolaldéhyde, le 3-acétylcarbazole, le 3,6-diacétyl-9-éthylcarbazole, le 3-acétyl-9-méthylcarbazole, le 1,4-diméthyl-3-carbazolaldéhyde, le 1,4,9-triméthyl-3-carbazolaldéhyde,
l'halogénure, le benzènesulfonate, le p-toluènesulfonate, l'alcane(en C₁-C₄)sulfonate, l'alkyl(en C₁-C₄)sulfate, le trifluorométhanesulfonate, le perchlorate, le sulfate, l'hydrogénosulfate, le tétrafluoroborate, le phosphate, l'hexafluorophosphate ou le tétrachlorozincate de 4-triméthylammoniobenzaldéhyde, de 4-benzyldiméthylammonio-benzaldéhyde, de 4-triméthylammoniocinnamaldéhyde, de 4-triméthylammonionaphtaldéhyde, de 2-méthoxy-4-triméthylammonio-benzaldéhyde, de 4-acétylphényltriméthylammonium, de 4-(N,N-diéthyl-méthylammonio)-benzaldéhyde, de 4-benzoylphényltriméthylammonium, de N-(4'-benzoylphényl)-N,N-diéthylméthylammonium, de N-(4'-formyl-phényl)-N-méthylpyrrolidinium, de N-(4'-formylphényl)-N-méthylpipéridinium, de N-(4'-formylphényl)-N-méthylmorpholinium, de N-(4'-acétylphényl)-N-méthylmorpholinium, de N-(4'-benzoylphényl)-N-méthylmorpholinium, de 3-formyl-N-éthyl-N-méthylcarbazolium, de 1-(4'-acétylphényl)-3-méthylimidazolium, de 1-(4'-acétylphényl)-3-méthyl-2-imidazolinium, de 1-(4'-benzoylylphényl)-3-méthylimidazolium, de 5-acétyl-1,3-diéthyl-2-méthylbenzimidazolium, de 5-triméthylammonio-1-indanone,
le benzènesulfonate, le p-toluènesulfonate, le méthanesulfonate, le perchlorate, le sulfate, le chlorure, le bromure, l'iodure, le tétrachlorozincate, le méthylsulfate, le trifluorométhanesulfonate, le tétrafluoroborate de 4-formyl-1-méthylpyridinium, de 2-formyl-1-méthylpyridinium, de 4-formyl-1-éthylpyridinium, de 2-formyl-1-éthylpyridinium, de 4-formyl-1-benzylpyridinium, de 2-formyl-1-benzylpyridinium, de 4-formyl-1,2-diméthylpyridinium, de 4-formyl-1,3-diméthylpyridinium, de 4-formyl-1-méthylquinolinium, de 2-formyl-1-méthylquinolinium, de 4-acétyl-1-méthylpyridinium, de 2-acétyl-1-méthylpyridinium, de 4-acétyl-1-méthylquinolinium, de 5-formyl-1-méthylquinolinium, de 6-formyl-1-méthylquinolinium, de 7-formyl-1-méthylquinolinium, de 8-formyl-1-méthylquinolinium, de 5-formyl-1-éthylquinolinium, de 6-formyl-1-éthylquinolinium, de 7-formyl-1-éthylquinolinium, de 8-formyl-1-éthylquinolinium, de 5-formyl-1-benzylquinolinium, de 6-formyl-1-benzylquinolinium, de 7-formyl-1-benzylquinolinium, de 8-formyl-1-benzylquinolinium, de 5-formyl-1-allylquinolinium, de 6-formyl-1-allylquinolinium, de 7-formyl-1-allylquinolinium et de 8-formyl-1-allylquinolinium, de 5-acétyl-1-méthylquinolinium, de 6-acétyl-1-méthylquinolinium, de 7-acétyl-1-méthylquinolinium, de 8-acétyl-1-méthylquinolinium, de 5-acétyl-1-éthylquinolinium, de 6-acétyl-1-éthylquinolinium, de 7-acétyl-1-éthylquinolinium, de 8-acétyl-1-éthylquinolinium, de 5-acétyl-1-benzylquinolinium, de 6-acétyl-1-benzylquinolinium, de 7-acétyl-1-benzylquinolinium, de 8-acétyl-1-benzylquinolinium, de 5-acétyl-1-allylquinolinium, de 6-acétyl-1-allylquinolinium, de 7-acétyl-1-allylquinolinium et de 8-acétyl-1-allylquinolinium, de 9-formyl-10-méthylacridinium, de 4-(2'-formylvinyl)-1-méthylpyridinium, de 1,3-diméthyl-2-(4'-formylphényl)-benzimidazolinium, de 1,3-diméthyl-2-(4'-formylphényl)imidazolinium, de 2-(4'-formylphényl)-3-méthylbenzothiazolium, de 2-(4'-acétylphényl)-3-méthylbenzothiazolium, de 2-(4'-formylphényl)-3-méthylbenzoxazolium, de 2-(5'-formyl-2'-furyl)-3-méthylbenzothiazolium, de 2-(5'-formyl-2'-furyl)-3-méthylbenzothiazolium, de 2-(5'-formyl-2'-thiényl)-3-méthylbenzothiazolium, de 2-(3'-formylphényl)-3-méthylbenzothiazolium, de 2-(4'-formyl-1-naphtyl)-3-méthylbenzothiazolium, de 5-chloro-2-(4'-formylphényl)-3-méthylbenzothiazolium, de 2-(4'-formylphényl)-3,5-diméthyl-benzothiazolium, de 1-méthyl-2-[2-(4'-formylphényl)éthényl]-pyridinium, de 1-méthyl-4-[2-(4'-acétylphényl)éthényl]pyridinium, de 1-benzyl-4-[2-(4'-formylphényl)éthényl]pyridinium, de 1-méthyl-4-[2-(4'-formylphényl)éthényl]pyridinium, de 1-méthyl-2-[2-(4'-formylphényl)-éthényl]pyridinium de 1-méthyl-4-[2-(4'-formylphényl)éthényl]quinolinium, de 1-méthyl-2-[2-(5'-formyl-2'-furyl)éthényl]quinolinium, de 1-méthyl-2-[2-(5'-formyl-2'-thiényl)éthényl]quinolinium, de 1-méthyl-2-[2-(4'-formylphényl)éthényl]benzothiazolinium, de 1,3-diméthyl-2-[2-(4'-formylphényl)-éthényl]benzimidazolinium, de 1,3-diméthyl-2-[2-(4'-formyl-phényl)-éthényl]imidazolinium, de 1-méthyl-5-oxo-indéno[1,2-b]pyridinium (4-méthyl-4-azonio-9-fluorénone), de 1-éthyl-5-oxo-indéno[1,2-b]pyridinium (4-éthyl-4-azonio-9-fluorénone), de 1-benzyl-5-oxo-indéno[1,2-b]pyridinium (4-benzyl-4-azonio-9-fluorénone), de 2-méthyl-5-oxo-indéno[1,2-c]pyridinium, de 2-méthyl-9-oxo-indéno[2,1-c]-pyridinium, de 1-méthyl-9-oxo-indéno[2,1-b]pyridinium,
l'isatine, la 1-méthyl-isatine, la 1-allyl-isatine, la 1-hydroxyméthyl-isatine, la 5-chloro-isatine, la 5-méthoxy-isatine, la 5-nitro-isatine, la 6-nitro-isatine, la 5-sulfo-isatine, la 5-carboxy-isatine, la chinisatine, la 1-méthylchinisatine,
l'acénaphtènequinone, le benzile, le 2,2'-furile, le 2,2'-pyridile, le 4,4'-pyridile, le salicile, l'anisile, ainsi que n'importe quels mélanges des composés repris ci-dessus.

3. Agent selon la revendication 1 ou 2, **caractérisé en ce qu'**on met en oeuvre, à titre de composés répondant à la formule IIa, respectivement IIb,
la 4-amino-2,3-diméthyl-1-phényl-3-pyrazolin-5-one (la 4-aminoantipyrine), la 4-amino-2,3-diméthyl-1-(4'-chlorophényl)-3-pyrazolin-5-one, la 4-amino-2,3-diméthyl-1-(2'-chlorophényl)-3-pyrazolin-5-one, la 4-amino-2,3-diméthyl-1-(4'-fluorophényl)-3-pyrazolin-5-one, la 4-amino-2,3-diméthyl-1-(4'-méthoxyphényl)-3-pyrazolin-5-one, la 4-amino-2,3-diméthyl-1-(p-tolyl)-3-pyrazolin-5-one, la 4-amino-2-éthyl-3-méthyl-1-phényl-3-pyrazolin-5-one, la 4-amino-2,3-diméthyl-1-(2',4'-dichlorophényl)-3-pyrazolin-5-one, la 4-amino-1,2-diphényl-3-hydroxy-3-pyrazolin-5-one, la 4-amino-1,2-bis-(4'-chlorophényl)-3-hydroxy-3-pyrazolin-5-one, la 4-amino-3-méthyl-1-phényl-2-pyrazolin-5-one, la 4-amino-3-méthyl-1-(4'-sulfophényl)-2-pyrazolin-5-one, la 4-amino-3-méthyl-1-(4'-méthoxyphényl)-2-pyrazolin-5-one, la 4-amino-3-méthyl-1-(4'-chlorophényl)-2-pyrazolin-5-one, la 4-amino-3-méthyl-1-(3'-chlorophényl)-2-pyrazolin-5-one, la 4-amino-3-carbamoyl-1-phényl-2-pyrazolin-5-one, la 4-amino-3-carboxy-1-phényl-2-pyrazolin-5-one, la 4-amino-3-éthoxycarboyl-1-phényl-2-pyrazolin-5-one, la 4-amino-1-phényl-2-pyrazolin-5-one, la 4-amino-1-(4'-chlorophényl)-2-pyrazolin-5-one, la 4-amino-1-(4'-fluorophényl)-2-pyrazolin-5-one, la 4-amino-1-(4'-chlorophényl)-2-pyrazolin-5-one, la 4-amino-1-(4'-méthoxyphényl)-2-pyrazolin-5-one, la 4-amino-1-(4'-carboxyphényl)-2-pyrazolin-5-one, ainsi que leurs produits d'addition d'acides compatibles avec la peau.

4. Agent selon l'une quelconque des revendications 1 à 3, **caractérisé en ce que** les aldéhydes et/ou les cétones aromatiques ou hétéroaromatiques répondant à la formule I et les 4-aminopyrazolin-5-ones répondant à la formule IIa, respectivement IIb, sont contenus respectivement en une quantité de 0,03 à 65 millimoles, en particulier de 1 à 40 millimoles, rapportés à 100 g de la teinture totale.

5. Agent selon l'une quelconque des revendications 1 à 4, **caractérisé en ce qu'**il contient en outre, au moins un composé contenant des groupes hydroxyle ou des groupes amino primaires ou secondaires, choisi parmi le groupe comprenant des amines aromatiques primaires ou secondaires, des composés hétérocycliques azotés et des composés hydroxylés aromatiques, des acides aminés, des oligopeptides composés de 2 à 9 acides aminés et/ou au moins un composé contenant un groupe CH-acide et/ou un composé d'ammonium quaternaire.

6. Agent selon la revendication 5, **caractérisé en ce que** le composé supplémentaire est choisi parmi le groupe comprenant
des amines primaires ou secondaires choisies parmi le groupe constitué par la N-(2'-hydroxyéthyl)-N-éthyl-, la N-(2'-méthoxyéthyl)-, la 2,3-, la 2,4-, la 2,5-dichloro-p-phénylènediamine, la 2-chloro-p-phénylènediamine, la N,N-bis-(2'-hydroxyéthyl)-p-phénylènediamine, le dibromhydrate de la 2,5-dihydroxy-4-morpholino-aniline, le 2-, le 3-, le 4-aminophénol, la o-, la m-, la p-phénylènediamine, le 2,4-diaminophénoxyéthanol, le 2-(2',5'-diaminophényl)éthanol, le 2,5-diamino-toluène, -phénol, -phénéthol, la 4-méthylamino-, la 3-amino-4-(2'-hydroxyéthyloxy)-, la 3,4-méthylènediamino-, la 3,4-méthylènedioxyaniline, le 3-amino-2,4-dichloro-, le 4-méthylamino-, le 2-méthyl-5-amino-, le 3-méthyl-4-amino-, le 2-méthyl-5-(2'-hydroxyéthylamino)-, le 2-méthyl-5-amino-4-chloro-, le 6-méthyl-3-amino-2-chloro-, le 5-(2'-hydroxyéthylamino)-4-méthoxy-2-méthyl-, le 4-amino-2-aminométhylphénol, le 2-hydroxyméthyl-4-aminophénol, le 2-diéthylaminométhyl-4-aminophénol, le 2-diméthylaminométhyl-4-aminophénol, le 2,6-dichloro-4-aminophénol, le 1,3-diamino-2,4-diméthoxybenzène, l'acide 2-, l'acide 3-, l'acide 4-aminobenzoïque, l'acide 2-, l'acide 3-, l'acide 4-aminophénylacétique, l'acide 2,3-, l'acide 2,4-, l'acide 2,5-, l'acide 3,4-, l'acide 3,5-diaminobenzoïque, l'acide 4-, l'acide 5-aminosalicylique, l'acide 3-amino-4-hydroxy-, l'acide 4-amino-3-hydroxy-benzoïque, l'acide 2-, l'acide 3-, l'acide 4-aminobenzènesulfonique, l'acide 3-amino-4-hydroxybenzènesulfonique, l'acide 4-amino-3-hydroxy-naphtalène-sulfonique, l'acide 6-amino-7-hydroxynaphtalène-2-sulfonique, l'acide 7-amino-4-hydroxynaphtalène-2-sulfonique, l'acide 4-amino-5-hydroxy-naphtalène-2,7-disulfonique, l'acide 3-amino-2-naphtoïque, l'acide 3-aminophtalique, l'acide 5-amino-isophtalique, le 1,3,5-, le 1,2,4-triaminobenzène, le tétrachlorhydrate du 1,2,4,5-tétraaminobenzène, le trichlorhydrate du 2,4,5-triaminophénol, le pentachlorhydrate du pentaaminobenzène, l'hexachlorhydrate de l'hexaaminobenzène, le trichlorhydrate du 2,4,6-triaminorésorcinol, le sulfate du 4,5-diaminopyrocatéchol, le dichlorhydrate du 4,6-diaminopyrogallol, le sulfate du 3,5-diamino-4-hydroxypyrocatéchol, des nitriles aromatiques, des composés amino contenant un ou plusieurs groupes nitro, tels que la 3-amino-6-méthylamino-2-nitropyridine, l'acide picraminique, le chlorure de [8-[(4'-amino-2'-nitrophényl)azo]-7-hydroxy-napht-2-yl]-triméthyl-ammonium, le chlorure de [8-[(4'-amino-3'-nitrophényl)azo]-7-hydroxy-napht-2-yl]-triméthylammonium (Basic Brown 17), le 1-hydroxy-2-amino-4,6-dinitrobenzène, le 1-amino-2-nitro-4-bis-(2'-hydroxyéthyl)-aminobenzène, le 1-amino-2-(2'-hydroxyéthyl)amino-5-nitrobenzène (HC Yellow n° 5), le 1-amino-2-nitro-4-(2'-hydroxyéthyl)aminobenzène (HC Red n° 7), la 2-chloro-5-nitro-N-hydroxyéthyl-1,4-phénylènediamine, le 1-(2'-hydroxyéthyl)-amino-2-nitro-4-aminobenzène (HC Red n° 3), le 4-amino-3-nitrophénol, le 4-amino-2-nitrophénol, la 6-nitro-o-toluidine, le 1-amino-3-méthyl-4-(2'-hydroxyéthyl)-amino-6-nitrobenzène (HC Violet n° 1), le 1-amino-2-nitro-4-(2',3'-dihydroxypropyl)-amino-5-chlorobenzène (HC Red n° 10), l'acide 2-(4'-amino-2'-nitroanilino)benzoïque, la 6-nitro-2,5-diaminopyridine, le 2-amino-6-chloro-4-nitrophénol, le sel disodique de l'acide 1-amino-2-(3'-nitrophénylazo)-7-phénylazo-8-naphtol-3,6-disulfonique (Acid blue n° 29), le sel disodique de l'acide 1-amino-2-(2'-hydroxy-4'-nitrophénylazo)-8-naphtol-3,6-disulfonique (vert de palatinchrome), le sel disodique de l'acide 1-amino-2-(3'-chloro-2'-hydroxy-5'-nitrophénylazo)-8-naphtol-3,6-disulfonique (gallion), le sel disodique de l'acide 4-amino-4'-nitrostilbène-2,2'-disulfonique, le 2,4-diamino-3',5'-dinitro-2'-hydroxy-5-méthylazobenzène (Mordant brown 4), l'acide 4'-amino-4-nitrodiphénylamine-2-sulfonique, l'acide 4'-amino-3'-nitro-benzophénone-2-carboxylique, le 1-amino-4-nitro-2-(2'-nitro-benzylidènamino)-benzène, la 2-[2'-(diéthylamino)-éthylamino]-5-nitroaniline, l'acide 3-amino-4-hydroxy-5-nitrobenzènesulfonique (hydrate), le 3-amino-3'-nitrobiphényle, le 3-amino-4-nitro-acénaphtène, le 2-amino-1-nitronaphtalène, le 5-amino-6-nitrobenzo-1,3-dioxol, des anilines, en particulier des anilines contenant des groupes nitro, telles que la 4-nitroaniline, la 2-nitroaniline, le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2'-hydroxyéthylamino)-benzène, le 2-nitro-1-amino-4-[bis-(2'-hydroxyéthyl)-amino]benzène, l'acide 4-amino-2-nitrodiphénylamine-2'-carboxylique, le 1-amino-5-chloro-4-(2'-hydroxyéthylamino)-2-nitrobenzène, des anilines, en particulier des anilines contenant des groupes nitro, telles que la 4-nitroaniline, la 2-nitroaniline, le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2-hydroxyéthylamino)-benzène, le 2-nitro-1-amino-4-[bis-(2-hydroxyéthyl)-amino]benzène, l'acide 4-amino-2-nitrodiphénylamine-2'-carboxylique, le 1-amino-5-chloro-4-(2'-hydroxyéthylamino)-2-nitrobenzène, des anilines aromatiques, respectivement des phénols contenant un radical aromatique supplémentaire tel que le dichlorhydrate de 4,4'-diaminostilbène, le sel de sodium de l'acide 4,4'-diaminostilbène-2,2'-disulfonique, le 4,4'-diaminodiphénylméthane, le 4,4'-diaminodiphénylsulfure, le 4,4'-diaminodiphénylsulfoxyde, la 4,4'-diaminodiphénylamine, l'acide 4,4'-diaminodiphénylamine-2-sulfonique, la 4,4'-diaminobenzophénone, le 4,4'-diaminodiphényléther, le tétrachlorhydrate du 3,3',4,4'-tétra-aminodiphényle, la 3,3',4,4'-tétraaminobenzophénone, le tétrachlorhydrate du 1,3-bis-(2',4'-diaminophénoxy)propane, le tétrachlorhydrate du 1,8-bis-(2',5'-diaminophénoxy)-3,6-dioxaoctane, le 1,3-bis-(4'-aminophénylamino)propane, le 1,3-bis-(4'-aminophényl-amino)-2-propanol, le 1,3-bis-[N-(4'-aminophényl)-2-hydroxyéthylamino]-2-propanol, le trichlorhydrate de la N,N-bis-[2-(4'-aminophénoxy)-éthyl]-méthylamine,
des composés hétérocycliques azotés choisis parmi le groupe constitué par la 2-, la 3-, la 4-amino-, la 2-amino-3-hydroxy-, la 2,6-diamino-, la 2,5-diamino-, la 2,3-diamino-, la 2-diméthylamino-5-amino-, la 3-amino-2-méthylamino-6-méthoxy-, la 2,3-diamino-6-méthoxy-, la 3,5-diamino-2,6-diméthoxy-, la 2,4,5-triamino-, la 2,6-dihydroxy-3,4-diméthyl-pyridine, la 4,5,6-triamino-, la 2-hydroxy-4,5,6-triamino-, la 4-hydroxy-2,5,6-triamino-, la 2,4,5,6-tétraamino-, la 2-méthylamino-4,5,6-triamino-, la 2,4-, la 4,5-diamino-, la 2-amino-4-méthoxy-6-méthyl-pyrimidine, le 2,3,4-triméthylpyrrole, le 2,4-diméthyl-3-éthyl-pyrrole, le 3,5-diamino-pyrazole, -1,2,4-triazole, le 3-amino-, le 3-amino-5-hydroxy-pyrazole, la 2-, la 3-, la 8-aminoquinoléine, la 4-aminoquinaldine, l'acide 2-, l'acide 6-aminonicotinique, la 5-aminoisoquinoléine, le 5-, le 6-aminoindazole, le 5-, le 7-aminobenzimidazole, -benzothiazole, la 2,5-dihydroxy-4-morpholino-aniline, ainsi que des dérivés d'indole et d'indoline, tels que le 4-, le 5-, le 6-, le 7-aminoindole, le 4-, le 5-, 6-, le 7-hydroxyindole, le 5,6-dihydroxyindole, la 5,6-dihydroxyindoline, la 4-hydroxyindoline et des dérivés de l'hydroxypyrimidine, ainsi que respectivement parmi les sels physiologiquement acceptables de ces composés, formés avec des acides de préférence inorganiques,
des composés hydroxylés aromatiques choisis parmi le groupe constitué par le 2-, le 4-, le 5-méthylrésorcinol, le 2,5-diméthylrésorcinol, le résorcinol, le 3-méthoxyphénol, le pyrocatéchol, l'hydroquinone, le pyrogallol, la phloroglucine, l'hydroxyhydroquinone, le 2-, le 3-, le 4-méthoxy-, le 3-diméthylamino-, le 2-(2'-hydroxyéthyl)-, le 3,4-méthylènedioxy-phénol, l'acide 2,4-, l'acide 3,4-dihydroxy-benzoïque, -phénylacétique, l'acide gallique, l'acide 2,4,6-trihydroxybenzoïque, la 2,4,6-trihydroxyacétophénone, le 2-, le 4-chlororésorcinol, le 1-naphtol, le 1,5-, le 2,3-, le 2,7-dihydroxynaphtalène, l'acide 6-diméthylamino-4-hydroxy-2-naphtalènesulfonique, l'acide 3,6-dihydroxy-2,7-naphtalène-sulfonique,
des acides aminés choisis parmi le groupe comprenant la β-alanine, l'arginine, l'histidine, la tyrosine, la phénylalanine, la DOPA (dihydroxyphénylalanine), l'ornithine, la lysine, la proline et le tryptophane,
des oligopeptides choisis parmi le glutathion ou encore des oligopeptides que l'on obtient dans les hydrolysats du collagène, de la kératine, de la caséine, de l'élastine, de la protéine de soja, du gluten de froment ou de la protéine d'amande,
des composés CH-acides choisis parmi le groupe constitué par l'iodure du 1,2,3,3-tétraméthyl-3H-indolium, le p-toluènesulfonate du 1,2,3,3-tétraméthyl-3H-indolium, le méthanesulfonate du 1,2,3,3-tétraméthyl-3H-indolium, la 1,3,3-triméthyl-2-méthylène-indoline (base de Fischer), l'iodure du 2,3-diméthylbenzothiazolium, le p-toluènesulfonate du 2,3-diméthylbenzothiazolium, le p-toluènesulfonate du 1,2-diméthylnaphto[1,2-d]thiazolium, le p-toluènesulfonate du 1-éthyl-2-méthylnaphto[1,2-d]thiazolium, la rhodanine, l'acide rhodanine-3-acétique, l'iodure du 1-éthyl-2-quinaldinium, l'iodure du 1,4-diméthylquinolinium, l'iodure du 1-méthyl-2-quinaldinium, l'iodure du 1,4-diméthylquinolinium, l'acide barbiturique, l'acide thiobarbiturique, l'acide 1,3-diméthylthiobarbiturique, l'acide 1,3-diéthylthiobarbiturique, l'acide diéthylthiobarbiturique, l'oxoindole, le 3-indoxylacétate, la coumaranone, la 1-méthyl-3-phényl-2-pyrazolinone, l'indane-1,3-dione, l'indan-1-one, le 1-dicyanméthylène-indane,
des sels d'ammonium quaternaires tels que le chlorure, le bromure, l'iodure, l'hydrogénosulfate, l'(hémi)sulfate du tétraméthyl-, du tétraéthyl-, du tétrapropyl-, du tétrabutyl-, du benzyltriméthyl-ammonium ainsi que le Polyquaternium 10.

7. Agent selon la revendication 6, **caractérisé en ce que** le composé supplémentaire est choisi parmi le groupe constitué par la N-(2'-hydroxyéthyl)-N-éthyl-, la 2-chloro-p-phénylène-diamine, la N,N-bis-(2'-hydroxyéthyl)-p-phénylènediamine, le 4-aminophénol, le 2-amino-6-chloro-4-nitrophénol, la p-phénylènediamine, le 2-(2',5'-diaminophényl)-éthanol, le 2,5-diaminotoluène, la 3,4-méthylènedioxyaniline, le 2-amino-4-(2'-hydroxyéthylamino)anisole, le 2-(2',4'-diaminophénoxy)éthanol, le 3-amino-2,4-dichloro-, le 2-méthyl-5-amino-, le 3-méthyl-4-amino-, le 2-méthyl-5-(2'-hydroxyéthylamino)-, le 2-méthyl-5-amino-4-chloro-, le 6-méthyl-3-amino-2-chloro-, le 2-aminométhyl-4-amino-phénol, le 2-diéthylaminométhyl-4-aminophénol, le 2-diméthyl-aminométhyl-4-aminophénol, le 2,6-dichloro-4-aminophénol, le 2-hydroxyméthyl-4-aminophénol, le 3,4-méthylènedioxyphénol, l'acide 3,4-diaminobenzoïque, la 2,5-diamino-, la 2-diméthylamino-5-amino-, la 3-amino-2-méthylamino-6-méthoxy-, la 2,3-diamino-6-méthoxy-, la 3,5-diamino-2,6-diméthyl-pyridine, la 2,6-dihydroxy-3,4-diméthyl-pyridine, la 2-hydroxy-4,5,6-triamino-, la 4-hydroxy-2,5,6-triamino-, la 2,4,5,6-tétra-amino-, la 2-méthylamino-4,5,6-triamino-pyrimidine, le 3,5-diaminopyrazole, le 3-amino-5-hydroxypyrazole, le 4,5-diamino-1-(2-hydroxyéthyl)-pyrazole, le 5,6-dihydroxyindole et la 5,6-dihydroxyindoline, le bromure de tétra-butylammonium, la β-alanine, la L-proline, la L-lysine, la DL-tyrosine, ainsi que respectivement parmi les sels physiologiquement acceptables de ces composés, formés de préférence avec des acides inorganiques.

8. Agent selon l'une quelconque des revendications 1 à 7, **caractérisé en ce qu'**il contient des agents de renforcement de colorants choisis parmi le groupe constitué par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazidine ou l'un quelconque de leurs mélanges.

9. Agent selon l'une quelconque des revendications 1 à 8, **caractérisé en ce qu'**il contient des colorants montant directement sur la fibre choisis parmi le groupe comprenant des nitrophénylènediamines, des nitroaminophénols, des anthraquinones ou des indophénols, de préférence en une quantité de 0,01 à 20 % en poids rapportés à la teinture totale.

10. Agent selon l'une quelconque des revendications 1 à 9, **caractérisé en ce qu'**on ajoute des sels d'ammonium ou des sels métalliques choisis parmi le groupe comprenant des formiates, des carbonates, des halogénures, des sulfates, des butyrates, des valérates, des caproates, des acétates, des lactates, des glycollates, des tartrates, des citrates, des gluconates, des propionates, des phosphates et des phosphonates de métaux alcalins tels que le potassium, le sodium ou le lithium, de métaux alcalino-terreux tels que le magnésium, le calcium, le strontium ou le baryum ou encore de l'aluminium, du manganèse, du fer, du cobalt, du cuivre ou du zinc.

11. Agent selon l'une quelconque des revendications 1 à 10, **caractérisé en ce qu'**il contient des agents d'oxydation, en particulier du H₂O₂, en une quantité de 0,01 à 6 % en poids, rapportés à la solution d'utilisation.

12. Agent selon l'une quelconque des revendications 1 à 11, **caractérisé en ce qu'**il contient des agents tensioactifs anioniques, zwitterioniques ou non ioniques.

13. Utilisation d'une combinaison d'au moins un aldéhyde et/ou une cétone aromatique ou hétéroaromatique répondant à la formule I ou d'un de leurs sels physiologiquement acceptables 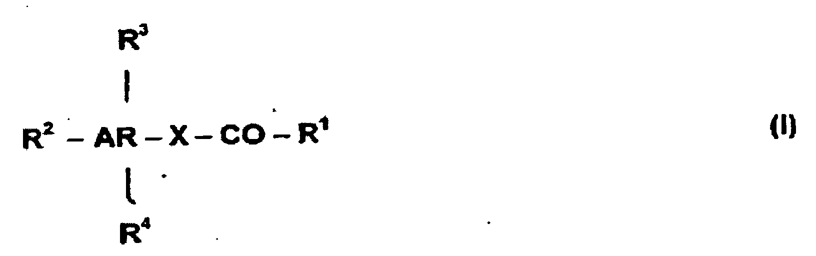 formule dans laquelle
• AR représente un groupe benzène, un groupe naphtalène, un groupe pyridine, un groupe pyrimidine, un groupe pyrazine, un groupe pyrazidine, un groupe carbazole, un groupe pyrrole, un groupe pyrazole, un groupe furanne, un groupe thiophène, un groupe 1,2,3-triazine, un groupe 1,3,5-triazine, un groupe quinoléine, un groupe isoquinoléine, un groupe indole, un groupe indoline, un groupe indolizine, un groupe indane, un groupe imidazole, un groupe 1,2,4-triazole, un groupe 1,2,3-triazole, un groupe tétrazole, un groupe benzimidazole, un groupe 1,3-thiazole, un groupe benzothiazole, un groupe indazole, un groupe benzoxazole, un groupe quinoxaline, un groupe quinazoline, un groupe quinolizine, un groupe cinnoline, un groupe acridine, un groupe julolidine, un groupe acénaphtène, un groupe fluorène, un groupe biphényle, un groupe diphénylméthane, un groupe benzophénone, un groupe diphényléther, un groupe azobenzène, un groupe chromone, un groupe coumarine, un groupe diphénylamine, un groupe stilbène, les composés hétéroaromatiques azotés pouvant également être quaternisés,
• R¹ représente un atome d'hydrogène, un groupe alkyle en C₁-C₄, un groupe acyle en C₁-C₅, un groupe alcényle en C₂-C₄, un groupe perfluoroalkyle en C₁-C₄, un groupe aryle ou hétéroaryle éventuellement substitué,
• R², R³ et R⁴ représentent, indépendamment l'un de l'autre, un atome d'hydrogène ; un atome d'halogène ; un groupe alkyle en C₁-C₄, un groupe alcoxy en C₁-C₄ ; un groupe aminoalkyle en C₁-C₄, un groupe hydroxyalkyle en C₁-C₄ ; un groupe acyle en C₁-C₄ ; un groupe carboxyle, un groupe carboxylato, un groupe sulfo, un groupe sulfato, un groupe alcényle en C₂-C₆, un groupe aryle ; un groupe arylalkyle en C₁-C₄ ; un groupe hydroxyle, un groupe nitro, un groupe pyrrolidino, un groupe morpholino, un groupe pipéridino, un groupe amino, respectivement un groupe ammonio ou un groupe 1-imidazol(in)io, les trois derniers groupes mentionnés pouvant être substitués avec un groupe alkyle en C₁-C₄, avec un groupe carboxyalkyle en C₁-C₄, avec un groupe hydroxyalkyle en C₁-C₄, avec des groupes alcényle en C₂-C₄, avec des groupes benzyle éventuellement substitués, avec des groupes sulfoalkyle en C₁-C₄ ou avec des groupes alkyle en C₁-C₄ contenant un noyau hétérocyclique, qui peuvent être substitués par un groupe alkyle en C₁-C₄, par un groupe alcényle en C₂-C₆ ou par un groupe hydroxyalcoxy en C₁-C₄, deux des radicaux choisis parmi le groupe comprenant R², R³, R⁴ et -X-CO-R¹ pouvant également former un noyau condensé à 5 membres, à 6 membres ou à 7 membres, éventuellement substitué, qui peut également porter un noyau aromatique condensé, le système AR, en fonction de la dimension du noyau pouvant porter d'autres substituants qui peuvent représenter, indépendamment les uns des autres, les mêmes substituants que ceux indiqués pour R², R³ et R⁴,
• X représente une liaison directe ; un groupe carbonyle ; un groupe carboxyalkylène en C₁-C₄; un groupe alcénylène en C₂-C₆, un groupe alcadiénylène en C₄-C₆, un groupe furylène, un groupe thiénylène, un groupe arylène, un groupe vinylènearylène, un groupe vinylènefurylène, un groupe vinylènethiénylène, ces groupes étant éventuellement substitués ; X peut également former, ensemble avec le groupe CO-R¹, un noyau à 5 membres, à 6 membres ou à 7 membres, éventuellement substitué,
et d'au moins une 4-aminopyrazolin-5-one répondant à la formule (IIa) ou à la formule (IIb) ou de ses sels physiologiquement acceptables, 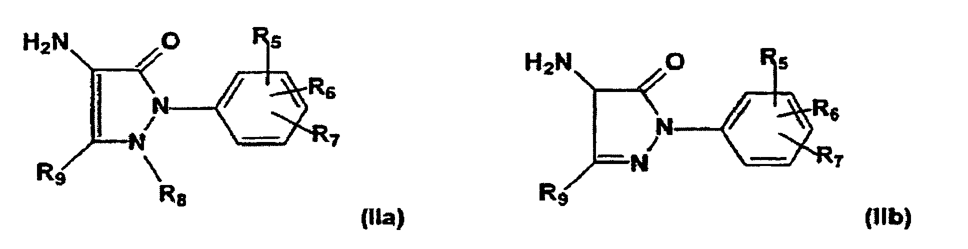 formules dans lesquelles
• R⁵, R⁶ et R⁷ représentent, indépendamment l'un de l'autre, un atome d'hydrogène, un atome d'halogène, un groupe alkyle en C₁-C₄, un groupe alcoxy en C₁-C₄ ou un groupe hydroxyalcoxy en C₁-C₄; un groupe hydroxyle, un groupe carboxyle, un groupe carboxylato, un groupe alcoxy(en C₁-C₄)carbonyle, un groupe carbamoyle éventuellement substitué, un groupe sulfo, un groupe sulfato, un groupe sulfamoyle ou un groupe amino, qui peut être substitué par des groupes alkyle ou hydroxyalkyle en C₁-C₄,
• R⁸ et R⁹ représentent, indépendamment l'un de l'autre, un atome d'hydrogène ; un groupe hydroxyle, un groupe amino, un groupe alkyle en C₁-C₄, un groupe alcoxy en C₁-C₄, un groupe carbamoyle, un groupe carboxyle, un groupe alcoxy(en C₁-C₄)-carbonyle, un groupe aryle, un groupe arylalkyle en C₁-C₄ ou un groupe hétéroaryle,
à titre d'un composant de coloration dans des teintures d'oxydation pour cheveux.

14. Procédé pour la teinture de fibres kératiniques, en particulier de cheveux humains, dans lequel on applique, sur les fibres kératiniques, une teinture contenant
A1) au moins un aldéhyde et/ou une cétone aromatique ou hétéroaromatique répondant à la formule I ou encore leurs sels physiologiquement acceptables 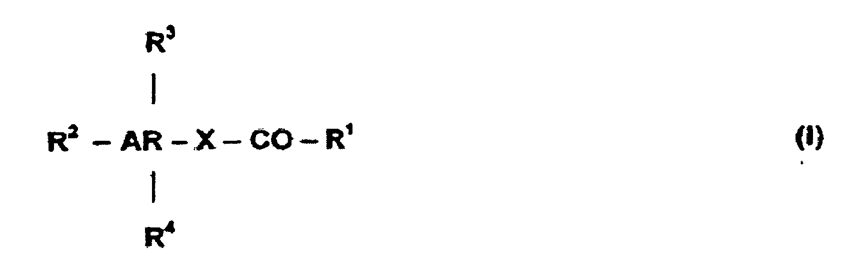 formule dans laquelle
• AR représente un groupe benzène, un groupe naphtalène, un groupe pyridine, un groupe pyrimidine, un groupe pyrazine, un groupe pyrazidine, un groupe carbazole, un groupe pyrrole, un groupe pyrazole, un groupe furanne, un groupe thiophène, un groupe 1,2,3-triazine, un groupe 1,3,5-triazine, un groupe quinoléine, un groupe isoquinoléine, un groupe indole, un groupe indoline, un groupe indolizine, un groupe indane, un groupe imidazole, un groupe 1,2,4-triazole, un groupe 1,2,3-triazole, un groupe tétrazole, un groupe benzimidazole, un groupe 1,3-thiazole, un groupe benzothiazole, un groupe indazole, un groupe benzoxazole, un groupe quinoxaline, un groupe quinazoline, un groupe quinolizine, un groupe cinnoline, un groupe acridine, un groupe julolidine, un groupe acénaphtène, un groupe fluorène, un groupe biphényle, un groupe diphénylméthane, un groupe benzophénone, un groupe diphényléther, un groupe azobenzène, un groupe chromone, un groupe coumarine, un groupe diphénylamine, un groupe stilbène, les composés hétéroaromatiques azotés pouvant également être quaternisés,
• R¹ représente un atome d'hydrogène, un groupe alkyle en C₁-C₄, un groupe acyle en C₁-C₅, un groupe alcényle en C₂-C₄, un groupe perfluoroalkyle en C₁-C₄, un groupe aryle ou hétéroaryle éventuellement substitué,
• R², R³ et R⁴ représentent, indépendamment l'un de l'autre, un atome d'hydrogène ; un atome d'halogène ; un groupe alkyle en C₁-C₄, un groupe alcoxy en C₁-C₄; un groupe aminoalkyle en C₁-C₄, un groupe hydroxyalkyle en C₁-C₄; un groupe acyle en C₁-C₄ ; un groupe carboxyle, un groupe carboxylato, un groupe sulfo, un groupe sulfato, un groupe alcényle en C₂-C₆, un groupe aryle ; un groupe arylalkyle en C₁-C₄ ; un groupe hydroxyle, un groupe nitro, un groupe pyrrolidino, un groupe morpholino, un groupe pipéridino, un groupe amino, respectivement un groupe ammonio ou un groupe 1-imidazol(in)io, les trois derniers groupes mentionnés pouvant être substitués avec un groupe alkyle en C₁-C₄, avec un groupe carboxyalkyle en C₁-C₄, avec un groupe hydroxyalkyle en C₁-C₄, avec des groupes alcényle en C₂-C₄, avec des groupes benzyle éventuellement substitués, avec des groupes sulfoalkyle en C₁-C₄ ou avec des groupes alkyle en C₁-C₄ contenant un noyau hétérocyclique, qui peuvent être substitués par un groupe alkyle en C₁-C₄, par un groupe alcényle en C₂-C₆ ou par un groupe hydroxyalcoxy en C₁-C₄, deux des radicaux choisis parmi le groupe comprenant R², R³, R⁴ et -X-CO-R¹ pouvant également former un noyau condensé à 5 membres, à 6 membres ou à 7 membres, éventuellement substitué, qui peut également porter un noyau aromatique condensé, le système AR, en fonction de la dimension du noyau pouvant porter d'autres substituants qui peuvent représenter, indépendamment les uns des autres, les mêmes substituants que ceux indiqués pour R², R³ et R⁴,
• X représente une liaison directe ; un groupe carbonyle ; un groupe carboxyalkylène en C₁-C₄; un groupe alcénylène en C₂-C₆, un groupe alcadiénylène en C₄-C₆, un groupe furylène, un groupe thiénylène, un groupe arylène, un groupe vinylènearylène, un groupe vinylènefurylène, un groupe vinylènethiénylène, ces groupes étant éventuellement substitués ; X peut également former, ensemble avec le groupe CO-R¹, un noyau à 5 membres, à 6 membres ou à 7 membres, éventuellement substitué,
A2) et au moins une 4-aminopyrazolin-5-one répondant à la formule (IIa) ou à la formule (IIb) ou ses sels physiologiquement acceptables, 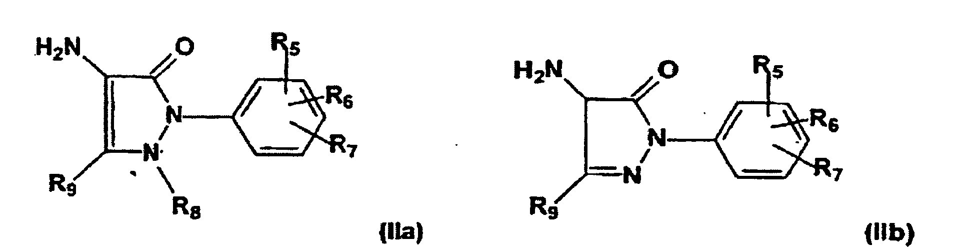 formules dans lesquelles
• R⁵, R⁶ et R⁷ représentent, indépendamment l'un de l'autre, un atome d'hydrogène, un atome d'halogène, un groupe alkyle en C₁-C₄, un groupe alcoxy en C₁-C₄ ou un groupe hydroxyalcoxy en C₁-C₄; un groupe hydroxyle, un groupe carboxyle, un groupe carboxylato, un groupe alcoxy(en C₁-C₄)carbonyle, un groupe carbamoyle éventuellement substitué, un groupe sulfo, un groupe sulfato, un groupe sulfamoyle ou un groupe amino, qui peut être substitué par des groupes alkyle ou hydroxyalkyle en C₁-C₄,
• R⁸ et R⁹ représentent, indépendamment l'un de l'autre, un atome d'hydrogène ; un groupe hydroxyle, un groupe amino, un groupe alkyle en C₁-C₄, un groupe alcoxy en C₁-C₄, un groupe carbamoyle, un groupe carboxyle, un groupe alcoxy(en C₁-C₄)-carbonyle, un groupe aryle, un groupe arylalkyle en C₁-C₄ ou un groupe hétéroaryle, et
B) éventuellement au moins un composé contenant des groupes hydroxyle ou des groupes amino primaires ou secondaires, choisi parmi le groupe comprenant des amines aromatiques primaires ou secondaires, des composés hétérocycliques azotés et des composés hydroxylés aromatiques, des acides aminés, des oligopeptides composés de 2 à 9 acides aminés et/ou au moins un composé contenant un groupe CH-acide ou un composé d'ammonium quaternaire,
ainsi que des constituants cosmétiques habituels, on les laisse un certain temps, habituellement pendant 30 minutes, sur les fibres, avant de les en éliminer par rinçage ou par lavage avec un shampooing.
